(19) Europäisches Patentamt
European Patent Office
Office européen des brevets

(11) EP 4 001 433 A1

(12) EUROPEAN PATENT APPLICATION

(43) Date of publication:
**25.05.2022 Bulletin 2022/21**

(21) Application number: **20306413.4**

(22) Date of filing: **20.11.2020**

(51) International Patent Classification (IPC):
***C12Q 1/686*** *(2018.01)* ***C12Q 1/6886*** *(2018.01)*

(52) Cooperative Patent Classification (CPC):
(C-Sets available)
**C12Q 1/6886; C12Q 1/686;** C12Q 2600/156;
C12Q 2600/16 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université de Paris**
  **75006 Paris (FR)**
- **Assistance Publique Hôpitaux de Paris**
  **75004 Paris (FR)**
- **Centre national de la recherche scientifique**
  **75016 Paris (FR)**
- **INSERM (Institut National de la Santé et de la Recherche Médicale)**
  **75013 Paris (FR)**
- **SORBONNE UNIVERSITE**
  **75006 Paris (FR)**

(72) Inventors:
- **POULET, Geoffroy**
  **75008 Paris (FR)**
- **NIZARD, Philippe**
  **75020 Paris (FR)**
- **TALY, Valérie**
  **92340 Bourg-la Reine (FR)**
- **LAURENT-PUIG, Pierre**
  **92190 Meudon (FR)**

(74) Representative: **Cabinet Becker et Associés**
**25, rue Louis le Grand**
**75002 Paris (FR)**

# (54) A METHOD FOR DETERMINING THE LEVEL OF DNA INTEGRITY

(57) The invention relates to a method for determining the level of integrity of DNA molecules in a DNA-containing sample by multiplexed digital PCR, which method comprises amplifying DNA fragments of the sample with amplification primers designed to produce different and predetermined sizes of overlapping amplicons of the same DNA target region, and oligonucleotide probes which can produce detectable and differential signals upon hybridization.

B

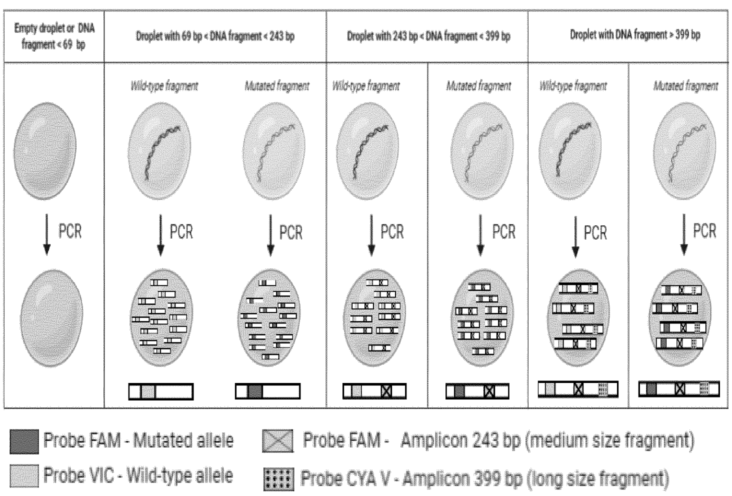


Figure 1 (cont.)



EP 4 001 433 A1

(52) Cooperative Patent Classification (CPC): (Cont.)

C-Sets
**C12Q 1/686**

## Description

### Technical background

Many methods have been developed for DNA integrity assessment including electrophoresis-based procedures, quantitative PCR, and, more recently, microfluidics-based procedures.

DNA integrity evaluation can be employed for characterizing biological samples quality before extensive genomic analysis. For instance the assessment of DNA integrity and quantity in a sample remains a bottleneck for different molecular genotyping technologies. In particular, DNA extracted from formalin-fixed paraffin-embedded (FFPE) tissue samples, a major potential source of tumor DNA, is subject to extensive fragmentation and damage that leads to unpredictable data quality.

Assessing DNA integrity also finds applications in reproductive medicine, prenatal diagnostics, and cancer research, as circulating DNA integrity can be used as a non-invasive biomarker in oncology.

There remains a need for sensitive and accurate methods for determining the level of integrity of a target DNA.

### Summary of the invention

The invention relates to an *in vitro* method for determining the level of integrity of DNA molecules in a DNA-containing sample by multiplexed digital PCR, which method comprises

a) amplifying DNA fragments of the sample with (i) amplification primers designed to produce at least n different and predetermined sizes of overlapping amplicons of the same DNA target region, and (ii) at least (n-1) oligonucleotide probes which can produce detectable and differential signals upon hybridization, wherein n is $\geq 2$ and each probe anneals to a distinct size of amplicon or to a combination of said amplicons,

and b) quantifying the DNA fragments of different sizes that are amplified, based on the signals from the oligonucleotide probes.

In a preferred embodiment, n is 2, 3, 4 or 5, preferably 3 or 4.

According to a preferred aspect of the invention, the DNA fragments are advantageously amplified by means of one forward (or reverse) and a set of at least n reverse (or, conversely forward) primers, wherein the melting temperatures (Tm) and/or the lengths of the primers of said set vary depending on the length of the DNA fragment to amplify.

The method is particularly useful in determining whether a subject is affected with a cancer. In such circumstances, the DNA containing sample is a biological sample from the subject, preferably a biological sample that contains circulating cell-free DNA (ccfDNA).

In a particular embodiment, the DNA target region comprises at least one marker associated with a disease or disorder, or is from at least one gene of interest, e.g. a housekeeping gene.

In a particular embodiment, the present invention provides an approach for accurate one-step assessment of DNA quantity and integrity from minute amounts of cell-free DNA using picoliter droplet digital PCR (ddPCR).

### Legends to the Figures

**Figure 1A** is a schematic drawing of the step of amplifying DNA fragments with one reverse primer and a set of 3 forward primers.

**Figure 1B** shows droplet based digital PCR multiplex assays amplifying different size of KRAS/BRAF wild-type (WT) and mutant (KRAS G13D / BRAF V600E) fragments (69 short, 235/243 medium and 394/399 long fragment). The methodology developed allows the monitoring of the DNA integrity and the discrimination of the mutated and wild-type allelic fraction (centered on a hotspot region).

**Figure 1C** shows fragmentation profile of wild-type (WT) gDNA extracted from commercial whole blood cell lines (purchased from Promega) (a), LoVo DNA (LoVo ATCC ® CCL-229™ (b) and HT29 DNA (HT-29 ATCC ® HTB-38™ (c). by using the "fragment > 399 bp" protocol of sonication (S220 Focused-Ultrasonicator sonicator; Covaris, Woburn, MA). DNA are fragmented between 50 bp and 700 bp.

**Figure 2A** is a schematic drawing of the region of KRAS (exon 2, codon 13) where the ccfDNA integrity assay was designed and validated.

**Figure 2B** shows a 2D histogram plot representing the analysis of fragmented and unfragmented DNA from LoVo cell line bearing KRAS G13D mutation.

**Figure 2C** shows a histogram representing repartition of the three fragment sizes according to whether they are derived from fragmented of unfragmented DNA from LoVo cell line.

**Figure 3A** is a schematic drawing of the region of BRAF (exon 12, codon 600), where the ccfDNA integrity assay was designed and validated.

**Figure 3B** shows a 2D histogram plot representing the analysis of fragmented and unfragmented DNA from HT29 cell line bearing BRAF V600E mutation.

**Figure 3C** shows a histogram representing repartition of the three fragment sizes in the tested samples according to whether they are derived from fragmented of unfragmented HT29 cell cell line.

**Figure 4** shows a comparison of KRAS and BRAF multiplex integrity assay on unfragmented **(A)** and fragmented **(B)** genomic DNA (commercial wild-type DNA extracted from whole blood).

**Figure 5** shows 2D histograms representing the analysis by multiplex integrity assay of DNA extracted from LoVo cell line purified by Blue Pippin [75-200 bp] **(A),** WT gDNA purified by Blue Pippin [75-200 bp] **(B),** WT gDNA purified by Blue Pippin [300-500 bp] **(C),** LoVo cell line purified by Blue Pippin [300-500 bp] **(D).**

**Figure 6** is a schematic drawing that shows the clinical workflow design to validate the multiplex integrity assay in biological sample and evaluation of the clinical relevance of an DNA integrity index as a biomarker for metastatic cancer.

**Figure 7A** shows a 2D histogram of results obtained with the multiplex integrity assay (Naica System software, STILLA Technologies platform) on healthy subjects.

**Figure 7B** shows a 2D histogram of results obtained with the multiplex integrity assay (Naica System software, STILLA Technologies platform) on mCRC (metastatic colorectal cancer) patients KRAS G13D.

**Figure 7C** represents the comparison of the ccfDNA integrity Index between healthy subjects, metastatic CRC patients and metastatic gastric patients using the multiplex integrity assay.

**Figure 8** shows a schema of the clinical workflow of the blood collection tube comparison study.

**Figure 9** shows the quantity of circulating DNA for n = 88 metastatic gastric samples determined by a fluorimetry technology (Qubit) and with the digital-droplet integrity test. A linear regression has been calculated to correlate the quantification data between the two technologies. DNA samples vary between 0.125 and 5.6 ng/mL.

**Figure 10** shows comparison of DNA extracted from plasma amount at day 0 for fragment > 399 bp, fragment 243-399 bp, fragment 69-243 bp and total DNA between LBgard® Blood Tubes and Cell-Free DNA BCT®.

**Figure 11** shows the evaluation of DNA extracted from plasma degradation between the two collection tubes (LBgard® Blood Tubes and Cell-Free DNA BCT®) over time after a 7-day tubes storage. Delta variation has been calculated for the two collection tubes and compared to each other for every DNA fragment, including total DNA.

**Figure 12** shows the evaluation of DNA extracted from plasma degradation between the two collection tubes (LBgard® Blood Tubes and Cell-Free DNA BCT®) over time at 10 days tubes storage. Delta variation has been calculated for the two collection tubes and compared to each other for each DNA fragment, including total DNA.

## Detailed description of the invention

*Definitions*

"Polymerase chain reaction" (PCR) refers to methods for increasing concentration of a segment of a target sequence in a mixture of genomic DNA without cloning or purification. The process for amplifying the target sequence includes introducing an excess of oligonucleotide primers to a DNA mixture containing a desired target sequence, followed by a precise sequence of thermal cycling in the presence of a DNA polymerase. The primers are complementary to their respective strands of the double stranded target sequence. To effect amplification, primers are annealed to their complementary sequence within the target molecule. Following annealing, the primers are extended with a polymerase so as to form a new pair of complementary strands. The steps of denaturation, primer annealing and polymerase extension can be repeated many times (i.e., denaturation, annealing and extension constitute one cycle; there can be numerous cycles) to obtain a high concentration of an amplified segment of a desired target sequence. The length of the amplified segment of the desired target sequence is determined by relative positions of the primers with respect to each other and by cycling parameters, and therefore, this length is a controllable parameter.

An "amplicon" refers to a nucleic acid fragment formed as a product of amplification events or techniques.

A mutation in a locus may be any form of substitution(s), deletion(s), rearrangements) and/or insertion(s) in the coding and/or non-coding region of the locus, alone or in various combination(s). Mutations more specifically include point mutations. Deletions may encompass any region of two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The mutation may result in the creation of stop codons, frameshift mutations, amino acid substitutions, particular RNA splicing or processing, product instability, truncated polypeptide production, etc. The alteration may result in the production of a polypeptide with altered function, stability, targeting or structure. The mutation may also cause a reduction in protein expression or, alternatively, an increase in said production.

As used herein, the term "subject" or "patient" refers to any vertebrate including, without limitation, humans and other primates (e.g., chimpanzees and other apes and monkey species), farm animals (e.g., cattle, sheep, pigs, goats and horses), domestic mammals (e.g., dogs and cats), laboratory animals (e.g., rodents such as mice, rats, and guinea pigs), and birds (e.g., domestic, wild and game birds such as chickens, turkeys and other gallinaceous birds, ducks, geese, and the like). In some implementations, the subject may be a mammal, preferably a human.

As used herein, a subject "susceptible to" a medical condition may alternatively be described as "is suspected to" or to "be considered at risk of being susceptible to "suffering or developing a medical condition; and in certain embodiments, the present invention is used to screen and/or diagnose the individual for susceptibility to, risk of suffering or developing, or suffering from or developing, a medical condition.

*The sample*

The methods of the invention may be implemented on any DNA containing sample.

In a preferred embodiment, the DNA containing sample is a biological sample, preferably a biological sample from a human or animal subject.

In a particular embodiment, the sample can be obtained from any cellular material, obtained from an animal, plant, bacterium, fungus, or any other cellular organism. In certain embodiments, the DNA molecules are obtained from a single cell. In certain embodiments, the sample is a tissue sample.

In a preferred aspect, the sample contains circulating cell-free DNA (ccfDNA), preferably the sample is a biological sample, preferably a body fluid, still preferably selected from the group consisting of serum, plasma, blood, urine, saliva, cerebrospinal fluid, pancreatic juice, duodenal fluids, peritoneal fluids. Other examples include sweat, ejaculate, teats, phlegm, vaginal secretion, vaginal wash and colonic wash.

In more particular embodiments, the sample is a plasma, serum or urine of a subject; in other embodiments, the sample is largely (or essentially) free from cells, and/or is not a whole blood and/or ejaculate sample.

Generally, the DNA can be extracted from the sample, e.g. by a variety of techniques such as those described by Maniatis, et al., Molecular Cloning: A Laboratory Manual, Cold Spring Harbor, N.Y., pp. 280-281 (1982).

*The DNA target region*

In the context of the present invention, the "DNA target region" refers to an individual locus in the genome that typically spans over between 200 and 1200 nucleotides, preferably over less than 1000 nucleotides, preferably less than 800 nucleotides, preferably less than 600 nucleotides, still preferably less than 400 nucleotides . In a preferred embodiment, the DNA target region is a single gene or a single regulatory element, *e.g.* a promoter.

In a particular embodiment, the DNA target region comprises at least one marker (e.g. an allele) associated with a disease or disorder.

The method of the invention is also particularly useful for assessing the quality of any DNA containing sample. In that prospect, the DNA target region preferably is a housekeeping gene. The term "housekeeping gene" as used herein is meant to refer to genes that encode protein products that are not connected to, involved in or required for processes specific to a disease state (e.g., cancer) in cells, and thus, exhibit a fixed expression level in diseased and healthy cells. Examples of suitable housekeeping genes include, but are not limited to, genes encoding ACTB (Beta-actin), GAPDH (Glyceraldehyde 3-phosphate dehydrogenase), RPLPO (60 S acidic ribosomal protein P0), GUSB (beta-glucuronidase), and TFRC (transferring receptor 1).

*The multiplexed digital PCR*

The method of the invention involves novel strategies for performing multiple different amplification reactions on the same sample simultaneously to quantify the abundance of multiple different amplicons, commonly known to those familiar with the art as "multiplexing". In one aspect, the invention provides materials and methods for improving amplicon yield while maintaining the sensitivity and specificity in digital PCR.

As used herein, "digital PCR" refers to an assay that provides an end-point measurement that provides the ability to quantify nucleic acids without the use of standard curves, as is used in real-time PCR. In a typical digital PCR experiment, the sample is randomly distributed into discrete partitions, such that some contain no nucleic acid template and others contain one or more template copies. The partitions are amplified to the terminal plateau phase of PCR (or end-point) and then read to determine the fraction of positive partitions. If the partitions are of uniform volume, the number of target DNA molecules present may be calculated from the fraction of positive end-point reactions using Poisson statistics, according to the following equation:

$$\lambda = -\ln(1-p) \quad (1)$$

wherein λ is the average number of target DNA molecules per replicate reaction and p is the fraction of positive end-point reactions. From λ, together with the volume of each replicate PCR and the total number of replicates analyzed, an estimate of the absolute target DNA concentration is calculated.

Digital PCR includes a variety of formats, including employing droplet digital PCR, BEAMing (beads, emulsion, amplification, and magnetic), microwell plates, bulk emulsion droplets, microfluidic compartments and nanoliter- or picoliter-scale droplets produced with microfluidics.

"Droplet digital PCR" (ddPCR) refers to a digital PCR assay that measures absolute quantities by counting nucleic acid molecules encapsulated in discrete, volumetrically defined, water-in-oil droplet partitions that support PCR amplification (Hindson, et al, 2011. Analytical Chemistry 83, 8604-8610; Pekin, et al, 2011, Lab on a Chip 11, 2156-66; Pinheiro, et al, 2012, Anal Chem 84, 1003-1011). A single ddPCR reaction may be comprised of at least 20,000 partitioned droplets per well.

A "droplet" or "water-in-oil droplet" refers to an individual partition of the droplet digital PCR assay. A droplet supports PCR amplification of template molecule(s) using homogenous assay chemistries and workflows similar to those widely used for real-time PCR applications (Hindson et al., 2011; Pekin et al., 2011; Pinheiro et al., 2012).
Droplet digital PCR may be performed using any platform that performs a digital PCR assay that measures absolute quantities by counting nucleic acid molecules encapsulated in discrete, volumetrically defined, water-in-oil droplet partitions that support PCR amplification. The strategy for droplet digital PCR may be summarized as follows: a sample is diluted and partitioned into thousands to millions of separate reaction chambers (water-in-oil droplets) so that each contains one or no copies of the nucleic acid molecule of interest. The number of "positive" droplets detected, which contain the target amplicon (i.e., nucleic acid molecule of interest), versus the number of "negative" droplets, which do not contain the target amplicon (i.e., nucleic acid molecule of interest), may be used to determine the number of copies of the nucleic acid molecule of interest that were in the original sample. Examples of droplet digital PCR systems include the QX200™ Droplet Digital PCR System by Bio-Rad, which partitions samples containing nucleic acid template into 20,000 nanoliter-sized droplets; the RainDrop™ digital PCR system by RainDance Technologies (today Biorad), which partitions samples containing nucleic acid template into 5,000,000 to 10,000,000 picoliter-sized droplets and the Crystal Digital PCR™ with the STILLA Naica® system which partitions samples containing nucleic acid template into 25,000 to 30,000 nanoliter-sized droplets.

Typically, the sample droplet may be pre-mixed with a primer or primers, or the primer or primers may be added to the droplet. In some embodiments, droplets created by segmenting the starting sample are merged with a second set of droplets including one or more primers for the target nucleic acid in order to produce final droplets. In embodiments involving merging of droplets, two droplet formation modules are used. In one embodiment, a first droplet formation module produces the sample droplets consistent with limiting or terminal dilution of target nucleic acid. A second droplet formation or reinjection module inserts droplets that contain reagents for a PCR reaction. Such droplets generally include the "PCR master mix" (known to those in the art as a mixture containing at least Taq polymerase, deoxynucleotides of type A, C, G and T, and magnesium chloride) and forward and reverse primers (known to those in the art collectively as "primers"), all suspended within an aqueous buffer. The second droplet also includes detectably labeled probes for detection of the amplified target nucleic acid, the details of which are discussed below. Different arrangements of reagents between the two droplet types is envisioned. For example, in another embodiment, the template droplets also contain the PCR master mix, but the primers and probes remain in the second droplets. Any arrangement of reagents and template DNA can be used according to the invention.
In a particular embodiment, the DNA molecules of the sample are encapsulated into droplets or compartments along with the primers and probes.
According to the invention, all amplification reactions on the DNA target region occur in the same droplet or compartment.

*The primers*

The primers are designed to amplify target DNA sequences that i) overlap and ii) have n predetermined sizes (n being an integer number).

The overlapping amplicons of n different and predetermined sizes typically are

- fragments having less than 150, preferably less than 120, less than 110, less than 80, less than 70 nucleotides, e.g. between 20 and 150 nucleotides, between 30 and 120 nucleotides, between 40 and 110 nucleotides, between 50 and 80 nucleotides ("short fragments");

- fragments having from 200 to 250 about nucleotides ("medium fragments"); and
- fragments having more than 300, preferably more than 380, 400, 450, 500, 550 or 600 nucleotides ("long fragments").

More generally, the n predetermined sizes differ by at least 40 nucleotides, preferably at least 50 nucleotides, still preferably at least 60, 70, 80, 90, 100, 150, 200, 250, 300, 350 or 400 nucleotides, but by no more than 1000, 900, 800, or 700 nucleotides.

According to the invention, the DNA fragments are amplified by means of one forward (or reverse) and a set of at least n reverse (or, conversely forward) primers, wherein the melting temperatures (Tm) and/or the lengths of the primers of said set vary depending on the length of the DNA fragment to amplify.

Primers can be prepared by a variety of methods including direct chemical synthesis using methods well known in the art. Primers can also be obtained from commercial sources. The lengths of the primers can be extended or shortened at the 5' end or the 3' end to produce primers with desired melting temperatures. Also, the annealing position of each primer pair can be designed such that the sequence and, length of the primer pairs yield the desired melting temperature. The simplest equation for determining the melting temperature of primers smaller than 25 base pairs is the Wallace Rule (Td=2(A+T)+4(G+C)). Another method for determining the melting temperature of primers is the nearest neighbor method. Computer programs can also be used to design primers, including but not limited to Array Designer Software (Arrayit Inc.), Oligonucleotide Probe Sequence Design Software for Genetic Analysis (Olympus Optical Co.), NetPrimer, and DNAsis from Hitachi Software Engineering. The Tm (melting or annealing temperature) of each primer is calculated using software programs such as Oligo Design, available from Invitrogen Corp.

In a preferred embodiment, the n primers of the set of at least n reverse (or, conversely forward) primers are designed so that the Tm temperatures between two primers differ by 2°C to 10°C, preferably 2°C to 8°C, still preferably 2°C to 6°C, still preferably 2°C to 4°C, and the difference between the maximum Tm and the minimum Tm of said n primers is at most 10°C.

For instance, when n is at least 3 and the DNA fragments that are to be quantified comprise or

- fragments having less than 150, preferably less than 80 nucleotides ("short fragments");
- fragments having from 200 to 250 about nucleotides ("medium fragments"); and
- fragments having more than 300, preferably more than 380, nucleotides ("long fragments"). the Tm of the primers of said set are then preferably as follows:

  the Tm of the primer that allows amplification of said long fragments equals the Tm of the primer that allows amplification of said medium fragments, minus 2°C to 6°C;
  and the Tm of the primer that allows amplification of said medium fragments equals the Tm of the primer that allows amplification of said short fragments, minus 2°C to 6°C.

*The probes*

Amplicons are detected using detectably labeled probes.

Within the context of this invention, a probe refers to a polynucleotide sequence which is complementary to and capable of specific hybridization with a target DNA sequence. Probes typically comprise single-stranded nucleic acids of between 8 to 30, preferably 10 to 25, more preferably of between 15 to 20. In one aspect, the method of the invention employs a nucleic acid probe specific for an altered (mutated) sequence, i.e., a nucleic acid probe that specifically hybridizes to said mutated sequence. Specificity indicates that hybridization to the target sequence generates a specific signal which can be distinguished from the signal generated through non-specific hybridization. Perfectly complementary sequences are preferred to design probes according to this invention. It should be understood, however, that certain mismatch may be tolerated, as long as the specific signal may be distinguished from non-specific hybridization.

In particular embodiments, the detectably labeled probes are optically labeled probes, such as fluorescently labeled probes.

Background fluorescence may be reduced to a minimum level, by maximizing self-quenching of the probe or to completely transfer the emitted light of the 5'-fluoresence donor to the 3'-acceptor, respectively. Self-quenching of qPCR probes can be further increased by equipping the probe internally with an additional quencher in optimal distance from the fluorescence donor. These kinds of probes are called double-quenched probes.

In certain aspects, the label on the oligonucleotide probe is a fluorescent label and the label is preferably selected from the group of FAM (5- or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor PET, Biosearch Blue™, Marina Blue®, Bothell Blue®, Alexa Fluor®, 350 FAM™, SYBR® Green 1, Fluorescein, EvaGreen™, Alexa Fluor® 488 JOE™, VIC™, HEX™, TET™, CAL Fluor® Gold 540, Yakima Yellow®, ROX™, CAL Fluor® Red 610, Cy3.5™, Texas Red®, Alexa Fluor®.568 Cy5™, Quasar™ 670, LightCycler Red640®, Alexa Fluor 633 Quasar™ 705, LightCycler Red705®, Alexa Fluor® 680, SYTO®9, LC Green®, LC Green® Plus+, EvaGreen™ fluorescent labels (i.e., dyes).

Labels other than fluorescent labels are contemplated by the invention, including other optically-detectable labels.

In certain aspects, the droplets or compartments contain a plurality of detectable probes that hybridize to amplicons produced. Members of the plurality of probes can each include the same detectable label, or a different detectable label. The plurality of probes can also include one or more groups of probes at varying concentration.

The invention preferably makes use of hydrolysis probes (TaqMan) but all DNA hybridization probes may be used, such as molecular beacons, Solaris probes, scorpion probes, and any other probes that function by sequence specific recognition of target DNA by hybridization and result in increased fluorescence on amplification or presence of the target sequence.

MGB (Minor Groove Binder) or Locked nucleic acid (LNA) containing probes can be used.

In a particular embodiment, at least one of the probes is designed to specifically anneal to a DNA sequence that carries a predetermined mutation. Such predetermined mutation includes allelic variants, insertion, deletion or substitution of one or several bases, e.g. single nucleotide polymorphisms (SNPs).

*Determining the level of DNA integrity*

The level of "DNA integrity" or "integrity of a target DNA" refers to the level of fragmentation of the DNA. "Determining the level of integrity of DNA molecules in a DNA-containing sample" thus involves quantifying the amounts of amplifiable copies of the target DNA.

A "DNA Integrity Index" (DII) may be determined, which is calculated e.g. as a ratio of the number of amplifies copies of longer fragments to shorter fragments. In the example below, the DII are analyzed using 302 bps amplicon (corresponding to the long fragments) and 60 bps, 84 bps and 184 bps amplicons as short and intermediate fragments.

The identification of the different fragment sizes is favored by the property of PCR efficiency. The "PCR efficiency" represents, at each PCR cycle, the capacity of the polymerase enzyme to double the amount of the target DNA region (amplicon). When a PCR efficiency is closed to 100%, at each PCR cycle, the target DNA region is duplicated. Nevertheless, PCR efficiency has been described to decrease when long DNA fragments are amplified. Therefore, in each PCR cycle, the number of amplicons generated is lower inside the droplets or compartments which encapsulate long DNA fragments rather than the droplets or compartments that comprise short DNA fragments. For instance, according to the initial DNA fragment incorporated inside the droplet or compartment, the number of hydrolyzed probes at the end of the $2^{50}$ PCR cycles is not the same and is lower for the long DNA fragments compared to the short DNA fragments. For this reason, the amplitude signal is higher for short DNA fragments than long DNA fragments and allows the discrimination of the different fragment sizes.

*Applications*

The method of the invention finds utility in a variety of fields.

In one aspect, the present invention provides an accurate and precise digital PCR approach to practically implement quality assurance for clinical cell-free DNA studies.

Accurate quantification and assessment of integrity is achievable using picogram amounts of cell-free DNA. DNA integrity and quantity assessments are predictive of library diversity and obtainable depth-of-coverage in next-generation sequencing libraries made from cell-free DNA samples.

In another aspect, the invention is useful in the diagnosis, prognosis and/or monitoring of medical conditions that involve a mechanism of DNA fragmentation, especially when the DNA species is circulating cell-free DNA and, more preferably, when said sample is a blood fraction such as plasma or serum. For example, the medical condition may be a cell proliferative disorder, such as a tumor or cancer. In particular embodiments, the medical condition is a tumor or a cancer of an organ selected from the list consisting of: liver, lung, breast, colon, esophagus, prostate, ovary, cervix, uterus, testis, brain, bone marrow and blood; and/or said species of DNA may originate from cells of a tumor; particularly where such tumor is a carcinoma or cancer of an organ selected from the group consisting of: liver, lung, breast, colon, esophagus, prostate, stomach, ovary, cervix, uterus, testis, brain, bone marrow, skin and blood.

In yet another particular embodiment of the present invention, said species of DNA originates from a cell type associated with a medical condition selected from the group consisting of: an infection/infectious disease, a wasting disorder, a degenerative disorder; an (auto)immune disorder, kidney disease, liver disease, inflammatory disease, acute toxicity, chronic toxicity, myocardial infarction, and a combination of any of the forgoing (such as sepsis) and/or with a cell proliferative disorder, particularly in those embodiments where said species of DNA is circulating cell-free DNA and said sample is a blood fraction such as plasma or serum.

The present invention is further illustrated by the following examples that should not be construed as limiting.

## EXAMPLES

### Example 1: Development of multiplex integrity assay targeting in the same reaction well, 3 different fragments sizes and identifying the mutational status of the DNA in a specific hotspot region

### 1.1. Materials and methods

Assay design: **(Figures 1A and 1B)**

Primers were designed to amplify preferentially the large DNA fragments, which are more difficult to amplify by PCR compared to short DNA fragments. The differentiation of DNA fragments sizes is based on the addition of Taqman-MGB probes on the medium (1 probe labelled with fluorochrome FAM) and the long fragment (1 probe labelled with fluorochrome FAM + 1 probe labelled with fluorochrome CYA V).

The allelic status (mutated or not mutated) at a specific hotspot region (BRAF V600E; KRAS G13D encoding gene) was differentiated based on a couple of probes (FAM fluorochrome for mutated sequence; VIC fluorochrome for wild-type sequence).

Digital-droplet PCR assay description

PCR reactive mixture was prepared as previously described. 10 ng of cell line DNA or WT gDNA or plasmatic DNA were mixed with assay mix with a final concentration of 1.4 μM of anti-sense primers, 1.3 μM sense primer (394 - 399 fragment), 0.76 μM sense primer (235-243 fragment) and 0.6 μM (69 fragment) and 0.3 μM of TaqMan® probes 1/2, 0.4 μM probe 3 and 0.7 μM probe 4, prior to encapsulation. Sequences for each multiplex integrity test (*KRAS* and *BRAF*) are described in Tables 1 and 2 respectively. All samples were thermal cycled using the Naica System touch. See Table 3. Naica system main settings to detect probes signal are provided in Table 4.

Table 1: Sequences and concentrations of Taqman® Probes and primers used for the KRAS multiplex integrity assay. Taqman® probes include MGB (minor groove binder) functionality with NFQ (non-fluorescent quencher) as a quencher.

| Targeted sequences | Reporter dye | Probe Sequence (5'>3') | Forward Primer sequence (5'>3') | Reverse Primer sequence (5'>3') |
|---|---|---|---|---|
| KRAS exon 2, codon 13 (WT sequence) | VIC | SEQ ID N°1: AGGGTTTTTATA ATTTA | | SEQ ID N°8: TGTATCGTCAAGG CACTC |
| KRAS exon 2 codon 13 (mutated sequence) | FAM | SEQ ID N°2: CGGCGTTAGGT TGC | | Same reverse primer |
| KRAS, exon 2, fragment 69 bp | - | No added probe | SEQ ID N°5: GAAAATGACTG AATATAAACTT GTGGTA | Same reverse primer |
| KRAS, exon 2, Fragment 243 bp | FAM | SEQ ID N°3: AAGGTACTGGT GGAGTAT | SEQ ID N°6: GCAGTCAACTG GAATTTTCA | Same reverse primer |
| KRAS, exon 2, Fragment 399bp | Cy5 | SEQ ID N°4: ACGTCTGCAGT CAACT | SEQ ID N°7: TGAGAGCCTTT AGCCG | Same reverse primer |

Table 2: Sequences and concentrations of Taqman® Probes and primers used for the BRAF multiplex integrity assay. Taqman® probes include MGB (minor groove binder) functionality with NFQ (non-fluorescent quencher) as a quencher.

| Targeted sequences | Reporter dye | Probe Sequence (5'>3') | Forward Primer sequence (5'>3') | Reverse Primer sequence (5'>3') |
|---|---|---|---|---|
| BRAF exon 15, codon 600 (WT sequence) | VIC | SEQ ID N°9: GGTCTAGCTA CAGAGAAATC | | SEQ ID N°16: CTGATGGGACCC ACTCC |
| BRAF exon 15, codon 600 (mutated sequence) | FAM | SEQ ID N°10: GGTCTAGCTA CAGTGAAAT | | Same reverse primer |
| BRAF, exon 15, fragment 69 bp | - | No adding probe | SEQ ID N°13: GACCTCACAG TAAAAATAGG TGATT | Same reverse primer |
| BRAF, exon 15, Fragment 235 bp | FAM | SEQ ID N°11: TGCTTGCTCTG ATAGGA | SEQ ID N°14: GACTCTAAGA GGAAAGATGA AG | Same reverse primer |
| BRAF, exon 15, Fragment 394 bp | Cy5 | SEQ ID N°12: TTAGGAAAGC ATCTCACC | SEQ ID N°15: TCTGGGCCTA CATTGC | Same reverse primer |

Table 3: PCR program for the multiplex integrity assay using Naica System (STILLA Technologies).

| PCR program | Temperature (C°) | Time | Cycle Numbers |
|---|---|---|---|
| Droplet generations (Sapphire VI chips) | 40 | 30' | 1 |
| Enzyme mix preparation | 95 | 3' | 1 |
| DNA denaturation | 95 | 15" | 50 |
| Hybridization and primer elongation | 58.5 | 1' 15" | 50 |
| Elongation finalization | 72 | 3' | 1 |
| Depressurization/Program closure | - | 30" | 1 |

Table 4: The Naica™Prism3 optical specifications and examples of compatible fluorophores

| | Blue Channel | Green Channel | Red Channel |
|---|---|---|---|
| Excitation range | 415-480 nm | 530-550 nm | 630-640 nm |
| Emission range | 495-515 nm | 560-610 nm | 655-720 nm |

(continued)

| | Blue Channel | Green Channel | Red Channel |
|---|---|---|---|
| Compatible fluorophores (reporter) | FAM, Fluorescein | Cy3, HEX, VIC | Alexa Fluor 647, Cy5 |

Within TaqMan® assay, probe 1 labelled with VIC-fluorophore (λex 538 nm / λem 554 nm) was designed to be specific to the WT allele, while probe 2 labelled with FAM fluorophore (λex 494 nm / λem 518 nm) was able to specifically hybridize to the mutated sequence(s) (p.G13D for *KRAS,* p.V600E for *BRAF*). Probe 3 labelled with FAM-fluorophore λex 494 nm / λem 518 nm) was designed to be specific to the medium size (235-243 bp) and long size (394-399 bp) fragments. The probe 4 bearing Cyanine 5-fluorophore (λex 678/ λem 694 nm) was used to identify only the long fragment (< 394 bp).

Cell lines, culture conditions, DNA extraction

Colorectal cancer cell lines LoVo and HT29 (KRAS-mutated, c.38G>A, p.G13D and *BRAF*-mutated, c,1919T>A, p.V600E) were purchased from ATCC (Manassas, VA 20110, US) and respectively cultured in F-12 medium (Thermo Fisher Scientific) and McCoy's 5A Medium (ATCC®), supplemented with 10% foetal bovine serum and 1% Sodium Pyruvate and Hepes (Thermo Fisher Scientific). Cells were incubated at 37°C with 5% CO2.

Cell lines genomic DNA were extracted using the QIAamp® DNA Mini and blood Kit (QIAGEN, Hilden, Germany) according to the manufacturer's instructions and eluted into 200 μL of Tris 10 mM at pH 8 and stored at -20°C.

DNA Fragmentation

DNA from LoVo (bearing p.G13D KRAS mutation), WT gDNA (purchased from Promega) and HT29 (bearing p.V600E *BRAF* mutation) were fragmented by sonication (S220 Focused-Ultrasonicator sonicator; Covaris, Woburn, MA) to obtain fragments ranging from 50 bp to 700 bp. The different fragmentation protocols are mentioned in Table 5. The size distribution of fragmented DNA was confirmed by the miniaturized electrophoresis Caliper system, LabChip® GX/GXII Microfluidic system (Perkin-Elmer, Villebon-sur-Yvette, France) using DNA assay 5K reagent kit (Perkin-Elmer, Villebon-sur-Yvette, France).

Table 5: Fragmentation's protocol by sonication (S220 Focused-Ultrasonicator sonicator; Covaris, Woburn, MA) to obtain a range of fragment from 50 bps to 700 bps. Working DNA solution: concentration of 10 ng/μL, Volume 50-60 μL.

| *Parameters* | *Fragment < 150 bps* | *150 < Fragments < 399 bps* | *Fragment > 399 bps* |
|---|---|---|---|
| *Peak intensity* | 451 | 400 | 105 |
| *Dusty force* | 20 | 10 | 10 |
| *Cycle/burst* | 200 | 200 | 200 |
| *Time (second)* | 300 | 190 | 80 |
| *Temperature range (°C)* | 5-9 | 5-9 | 5-9 |

Purification DNA by fragments size: Generation of control

DNA from LoVo and WT gDNA fragmented by sonification were purified by miniaturized electrophoresis Blue Pippin (Sage Science, Beverly, MA) according to manufacturer's protocol. Highly fragmented DNA was used as control for short fragments, whereas low fragmented DNA was used as control for long fragmets. Two-size ranges for each DNA were purified: one sample with fragments ranging from 75 to 200bp (mentioned as [75-200 bp]) and one sample with fragments ranging from 300 to 500bp (mentioned as [300-500 bp]) by using respectively a 3% and a 2% agarose gel. The [75-200 bp] sample was used as a digital PCR control for the amplicon of 69bp whereas the sample [300-500 bp] was used as a control for the amplicon of 243bp. Non-Fragmented DNA from LoVo and WT gDNA was used as a control for the 399 bp fragment. The initial DNA amount into the Blue Pippin well was 150 μg for each purification.

## 1.2.Results

Design and validation of ddPCR assays for ccfDNA integrity analysis with the use of fragmented and non-fragmented cell line DNA and WT gDNA.

The inventors designed a new ccfDNA integrity assay to monitor 3 different fragment sizes (69 to 235/243 bp, 235/243 to 394/399 bp and > 394/399 bp) and to discriminate a mutational hotspot region. The assay has been validated on *KRAS,* exon 2, codon 13 **(Figure 2A)** and *BRAF,* exon 12, codon 600 regions **(Figure 3A).** The inventors used mechanically fragmented DNA (sonicated) and non-fragmented DNA from WT gDNA, LoVo and HT29 colorectal cell lines bearing wild-type and/or mutated *KRAS*-mutated, c.38G>A, p.G13D and *BRAF*-mutated, c.1919T>A, p.V600E. An example of results obtained on unfragmented and fragmented DNA is represented as a 2D histogram plot (LoVo cell line bearing *KRAS* G13D mutation **(Figure 2B)** and HT29 cell line bearing *BRAF* V600E mutation **(Figure 3B).** For fragmented DNA, the length of fragments, evaluated using the Caliper Lab Chip (see Material and methods), varied between 50-70 bp and 700-800 bp with higher amounts of long size fragments **(Figure 1C).** As the PCR efficiency decreased when a long amplicon is amplified, the inventors intentionally advantaged the amplification of the longest fragments. To analyse data, the size of the fragments (mutated and non- mutated) was quantified (counting droplets and converting into a concentration). To validate the assays targeting two different DNA hotspot regions, fragmented and non-fragmented LoVo and HT29 DNA have been run in triplicate **(Figures 2C and 3C).** Mutation allele frequency has been determined for both cell line DNA (66% for *KRAS* G13D in LoVo cell line and 30% for *BRAF* V600E in HT29 cell line). Moreover, as expected, a majority of long fragments are observed in unfragmented cell line DNA (approximately 94% for both tests) whereas on fragmented LoVo and HT29 DNA, a majority of short fragments are observed (55% and 61% respectively for LoVo and HT29 fragmented DNA).

To compare the two integrity assays, unfragmented and fragmented genomic DNA (bearing no mutation and following the same fragmentation program) **(Figure 1C)** have been run in triplicate with both tests. As expected, we observed a majority of long fragments characterized by the detection of 394/399 bp fragments in non-fragmented genomic DNA **(Figure 4A)** (respectively 92,87% and 95% on genomic with *KRAS* and *BRAF* multiplex integrity assay). Short and medium size DNA fragments represent a small ratio of unfragmented genomic DNA (4,27% and 2,15% of short DNA fragments; 2,87% and 2,23% of medium DNA fragments respectively with the *KRAS* and *BRAF* multiplex integrity assay). Turning to fragmented genomic DNA **(Figure 4B),** short DNA fragments are the most represented (84,2% and 78,8% respectively for *KRAS* and *BRAF* multiplex integrity assay), followed by the medium size DNA fragments (12,07% and 16,42%) and then by long DNA fragments (3,76% and 4,64%)

Validation of the different clusters of the *KRAS* multiplex integrity assay by selecting fragments using Blue Pippin

To validate the multiplex integrity assay, the inventors first fragmented the WT gDNA and LoVo DNA with different covaris protocol in order to have different fragmentation profiles **(Figure 4).** Then, the highly fragmented DNA was purified with the Blue Pippin to select fragment sizes of between 75 and 200 bp and analyzed by the multiplex integrity assay and by using the Caliper Lab Chip **(Figure 5A and 5B** respectively for LoVo DNA and WT gDNA). The same treatment was performed for the average fragmented DNA sample targeting fragment size of between 300 and 500 bp (**Figure 5C and 5D,** respectively for WT gDNA and LoVo DNA). The Caliper Lab Chip allowed us to validate our DNA manufactured (hand-made) control.

The results obtained with the multiplex integrity assay developed on the Naica system allowed us to clearly identify the different clusters and to validate this digital PCR assay.

**Example 2: A multiplex integrity assay to determine an integrity index on circulating cell-free (ccf) DNA**

## 2.1. Materials and methods

Clinical cohort

The clinical study workflow is shown on **Figure 6.**

*Plasma ccfDNA from CRC patients*

Patients suffering with metastatic colorectal cancer (stage IIIB et IV) bearing *KRAS* p.G13D (n=12) mutations and metastatic gastric patient (stage III) bearing KRAS Wild-type sequence (n= 22) were respectively selected from the prospective clinical study (AGEO RASANC study before chemotherapy and PLAGAST study for patients undergoing treatment).

Plasma from AGEO RASANC and PLAGAST study were collected in Cell-Free DNA BCT® Streck tubes and extracted with the use of a magnetic beads methods (Automated Maxwell, Maxwell® RSC ccfDNA Plasma Kit and Maxwell® RSC ccfDNA LV Custom kit (Promega)). The starting extraction volume was 1 mL and 4 mL respectively for AGEO RASANC and PLAGAST study. Before extraction, plasma has been centrifuged at 5000 rpm at room temperature for 15 minutes. Elution has been out in 60 μL volume according to manufacturer's instructions for all plasma samples. After extraction, DNA samples were stored at -20°C. Then, DNA quantification and integrity experiments were carried out using the multiplex integrity assay as described in Example 1.

Plasma ccfDNA from healthy individuals: control group

Plasma from 30 healthy individuals (healthy cohort 1) were purchased from the society BIOPREDIC International and collected in Cell-Free DNA BCT® | Streck tubes (Orgentec SASU). DNA was extracted with the use of the ccfDNA Plasma kit (Promega) by RSC Maxwell and eluted in 60 μL of elution buffer. This cohort was used as control cohort for the patients from AGEO RASANC study (patient cohort 1, see below).

The starting volume of extraction was 1mL. Before extraction, plasma has been centrifuged at 5000 rpm at room temperature for 15 minutes. Then, DNA quantification and integrity experiments were carried our using the multiplex integrity assay as described in Example 1. All the healthy individuals are non-smoking, not pregnant and without cancer or inflammation disease.

Data analysis

Data were analyzed according to the manufacturer's instructions (CrystalReader Software, STILLA technologies). Since the number of droplets reveals the quantity of amplifiable target DNA, the fraction of amplifiable DNA in samples could be determined. Starting from the same amount of DNA, amplified fragmented copies of long, medium, and short fragments were counted to calculate the DNA Integrity Index.

$$DNA\ Integrity\ Index\ (DII) = \frac{[Fragment\ 69 - 243\ bp]total, MT\ or\ WT\ allele}{[Fragment\ 243 - 399\ bp]total, MT\ or\ WT\ allele}$$

### 2.2. Results

Validation of the multiplex integrity assay on healthy subjects, mCRC patients and gastric patient: Calculation and comparison of the ccfDNA integrity Index between the 3 populations

After validation of the different clusters representing different fragment sizes, the inventors analysed healthy subjects, mCRC patients and gastric patients with the KRAS multiplex integrity assay. **Figures 7A and 7B** show 2D histogram of results obtained with the multiplex integrity assay (Naica System software, STILLA Technologies platform) on healthy subjects **(Figure 7A)** and mCRC patients *KRAS* G13D **(Figure 7B).** DNA fragments between 69-243 bp and between 243-399 bp were precisely quantified. Then, an integrity index for each subject or patient has been calculated according to the equation mentioned in materiel and method. Integrity index coefficient for each sub-population has been statistically compared.

**Figure 7C** shows that there are significant differences of DNA integrity index in function of the status healthy/metastatic (Wilcoxon-test, p-value = 0.0019 and 0.014 respectively between healthy subject and mCRC patient and healthy subject-gastric patient). Nevertheless, no significant difference of DNA integrity index is observed between the two populations of metastatic cancer patients. These results allowed us to validate the multiplex integrity assay on human circulating DNA.

## Example 3: Validation of the multiplex integrity assay on circulating cell-free DNA to follow-up the DNA quality after a time storage inside blood collection tubes: Toward the development of a quality DNA test

### 3.1. Materials and methods

Clinical cohort

The clinical study workflow is represented **Figure 8.**

The main goal of this study was to evaluate the stabilization performance of two blood collection tubes on ccfDNA and its tumoral fraction (ctDNA).

Twenty-two mGastric patients undergoing follow-up from the prospective cohort PLAGAST (Clinical Trials.gov NCT02674373) were included in this study. For each patient, 4 blood tubes (2 Cell-Free DNA BCT® Streck and 2 LBgard® Blood Tubes) were sampled at the same time. Each tube has been filled at a volume of 7 - 8 mL.

The cohort has been split equally into two groups. For one group (n patient = 11), one Cell-Free DNA BCT® Streck and one LBgard® Blood Tube were stored for 3 hours at room temperature before plasma preparation. The two remaining tubes were conserved at room temperature (20°C) for 7 days. For the second group, one Cell-Free DNA BCT® and one LBgard® Blood Tube were stored for 3 hours at room temperature (20°C) before plasma preparation and the two remaining tubes were stored at room temperature for 10 days.

Blood sample collection and storage

Blood was drawn from mGastric patients by venipuncture into x2 Cell-Free DNA BCT® (Streck) and x2 LBgard® Blood Tube (Biomatrica) by nurses at Digestive Oncology department of Hospital European Georges Pompidou (AP-HP, Paris, France). Blood was drawn to fill the tubes (~8 mL) as per the manufacturer's recommendations and brought back to the laboratory (UMRS1138, Cordeliers Research Center, Team 26, Université de Paris, Paris) for storage and processing.

The cohort has been split equally into two groups. For one group (n patient = 11), one Cell-Free DNA BCT® Streck and one LBgard® Blood Tube were stored for 3 hours at room temperature before plasma preparation. The two remaining tubes were stored at room temperature (20°C) for 7 days. For the second group, one Cell-Free DNA BCT® and one LBgard® Blood Tube were stored for 3 hours at room temperature (20°C) before plasma preparation. The last two tubes were stored at room temperature for 10 days.

After the storage period, for each tube, 4 mL of plasma was generated from matched blood samples with a two centrifugation steps: The first one at low-speed (1200g, 10 min, RT) and the second one at high-speed (8000g, 10 min, RT). The plasma samples were aliquoted in two cryotubes of 2 mL volume and stored at -80°C by Biobank in the lab.

CcfDNA extraction and DNA samples storage

For each plasma sample, DNA was extracted by using the AX6720 Maxwell® RSC ccfDNA LV Custom kit (Promega) using the Maxwell® RSC Instrument. This kit based on a magnetic beads' method is automatized and can extract DNA from large plasma volume (up to 4 mL). DNA extracted were eluted in 72 µL volume and stored inside a 1,5 mL LoBind® Tubes (Eppendorf) at - 20°C.

Total DNA, 69-243 bp, 243-399 bp and > 399 bp DNA fragments were quantified by using the KRAS multiplex integrity assay developed in Part 1. For each sample, 5 ng of DNA were input into the reactive ddPCR well reaction.

### 3.2. Results

Multiplex integrity assay: A quantitative tool

Circulating DNA has been quantified by fluorimetry Qubit (Invitrogen™ Qubit™ 2 Fluorometer) and KRAS multiplex integrity test for each plasma sample (n = 88). A linear regression model shows a correlation between the two distribution ($R^2$ = 0.97, Pearson's test) confirming the ability of the ddPCR to quantify very precisely the DNA amount in a sample **(Figure 9).**

Comparison of DNA amount at baseline (Day 0) between LBgard® Blood Tubes and Cell-Free DNA BCT®

Total DNA amount, DNA fragments between 69 bp and 243 bp, DNA fragments between 243 bp and 399 bp and DNA fragments > 399 bp have been quantified for each sample. A quantitative comparison of each DNA fragment, including total DNA has been carried out between the two preservative tubes at baseline (Day 0 post-blood sampling). **Figure 10** shows that no significant difference is observed between the two preservative collection tubes (Wilcoxon-test, p-value = 0.23 and 0.22 respectively between fragment > 399 bp and fragment 243-399 bp) and slight differences are shown for fragments 69-243 bp and total DNA amount (Wilcoxon-test, p-value = 0.011 and 0.046 respectively between fragment 69-243 bp and total DNA amount > 399 bp and fragment 243-399 bp). These results suggest that the two tubes contain an equivalent amount of circulating DNA and present an identical DNA fragmentation profile.

Evaluation of DNA degradation between the 2 collection tubes (LBgard® Blood Tubes and Cell-Free DNA BCT®) over time after a 7- and 10-days tubes storage

In order to compare the performance of the two tubes in term of conservation and stabilization of white blood cells, a delta variation coefficient, reflecting the differences of DNA quantity between day 0 and after 7 or 10 days post-sampling has been calculated for the two collection tubes. Delta variation coefficients for the two preservatives tubes have been compared for every DNA fragments, including total DNA.

**Figure 11** shows that no significant difference is observed between the two tubes after a 7 day storage at room temperature (Wilcoxon-test, p-value = 0.85; 0.85; 0.77; 0.56 respectively for Total DNA amount, 69 bp-243 bp fragment, 243-399 bp fragment and > 399 bp fragment.

**Figure 12** shows that no significant difference is observed between the two tubes after a 10 day storage at room temperature (Wilcoxon-test, p-value = 0.85; 0.39; 0.85; 0.92 respectively for Total DNA amount, 69 bp-243 bp fragment, 243-399 bp fragment and > 399 bp fragment.

## Claims

1. An *in vitro* method for determining the level of integrity of DNA molecules in a DNA-containing sample by multiplexed digital PCR, which method comprises

   a) amplifying DNA fragments of the sample with (i) amplification primers designed to produce at least n different and predetermined sizes of overlapping amplicons of the same DNA target region, and (ii) at least (n-1) oligonucleotide probes which can produce detectable and differential signals upon hybridization, wherein n is $\geq 2$ and each probe anneals to a distinct size of amplicon or to a combination of said amplicons,
   and b) quantifying the DNA fragments of different sizes that are amplified, based on the signals from the oligonucleotide probes.

2. The method of claim 1, wherein n is 2, 3, 4 or 5, preferably 3 or 4.

3. The method of claim 1 or 2, wherein the DNA fragments are amplified by means of one forward (or reverse) and a set of at least n reverse (or, conversely forward) primers, wherein the melting temperatures (Tm) and/or the lengths of the primers of said set vary depending on the length of the DNA fragment to amplify.

4. The method of any claim 3, wherein the n primers of said set are designed so that the Tm temperatures between two primers differ by 2°C to 10°C, preferably 2°C to 8°C, still preferably 2°C to 6°C, still preferably 2°C to 4°C, and the difference between the maximum Tm and the minimum Tm of said n primers is at most 10°C.

5. The method of any of claims 1 to 4, wherein the DNA fragments that are quantified comprise or consist of:

   - fragments having less than 150, preferably less than 80 nucleotides ("short fragments"); and
   - fragments having more than 300, preferably more than 380, nucleotides ("long fragments").

6. The method of claim 5, wherein n is at least 3, and the DNA fragments that are amplified comprise or consist of:

   - fragments having less than 150, preferably less than 80 nucleotides ("short fragments");
   - fragments having from 200 to 250 about nucleotides ("medium fragments"); and
   - fragments having more than 300, preferably more than 380, nucleotides ("long fragments").

7. The method of claim 6, wherein the Tm of the primers of said set are as follows:

   the Tm of the primer that allows amplification of said long fragments equals the Tm of the primer that allows amplification of said medium fragments, minus 2°C to 6°C;
   and the Tm of the primer that allows amplification of said medium fragments equals the Tm of the primer that allows amplification of said short fragments, minus 2°C to 6°C.

8. The method according to any of claims 1 to 5, wherein the DNA containing sample is a biological sample, preferably a biological sample from a human or animal subject.

**9.** The method of claim 6, wherein the sample contains circulating cell-free DNA (ccfDNA), preferably wherein the sample is a biological sample, preferably a body fluid, still preferably selected from the group consisting of serum, plasma, blood, urine, saliva, cerebrospinal fluid, pancreatic juice, duodenal fluids, peritoneal fluids.

**10.** The method according to any of claims 1 to 9, wherein the digital PCR is a multiplexed digital droplet PCR (ddPCR).

**11.** The method according to any of claims 1 to 10, wherein the DNA molecules of the sample are encapsulated into droplets or compartments with said primers and probes.

**12.** The method according to any of claims 1 to 11, wherein the DNA target region comprises at least one marker associated with a disease or disorder, or is from at least one gene of interest, e.g. a housekeeping gene.

**13.** The method according to any of claims 1 to 12, wherein at least one of the probes is designed to specifically anneal to a DNA sequence that carries a predetermined mutation.

**14.** Use of the method as defined in any of claims 1 to 13, in determining whether a subject is affected with a cancer, wherein the DNA containing sample is a biological sample from the subject, preferably a biological sample that contains circulating cell-free DNA (ccfDNA).

**15.** The use of the method according to claim 14, wherein the method further comprises determining whether the sample contains a DNA sequence that carries a predetermined mutation, wherein the mutation preferably is a tumor-associated allelic variant.

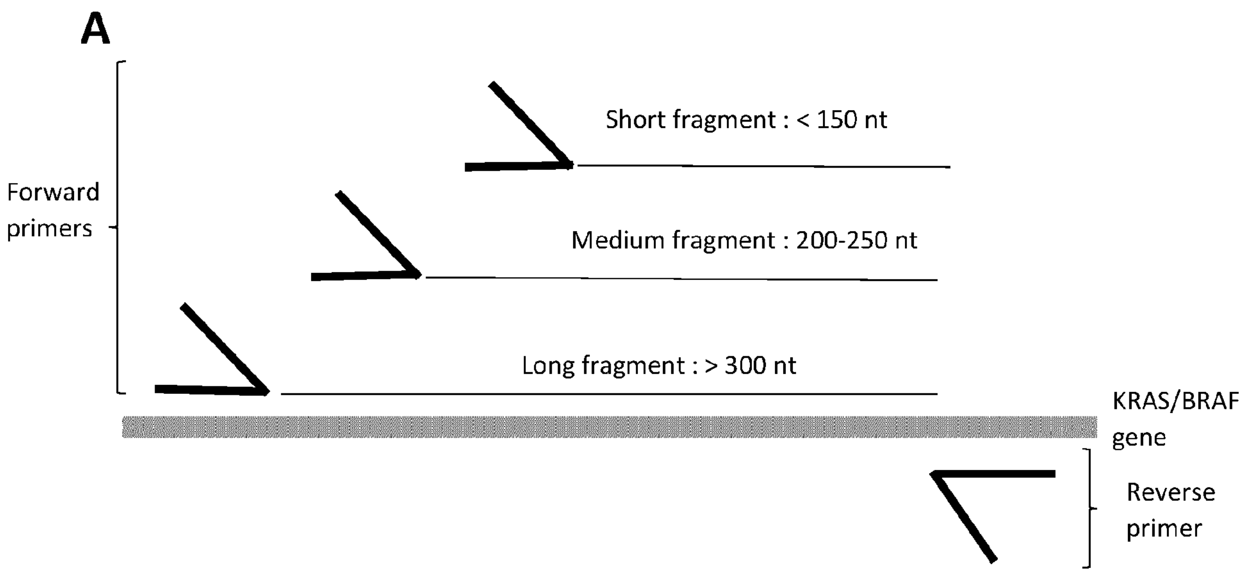
A
Short fragment : < 150 nt
Forward primers
Medium fragment : 200-250 nt
Long fragment : > 300 nt
KRAS/BRAF gene
Reverse primer

**Figure 1**

B

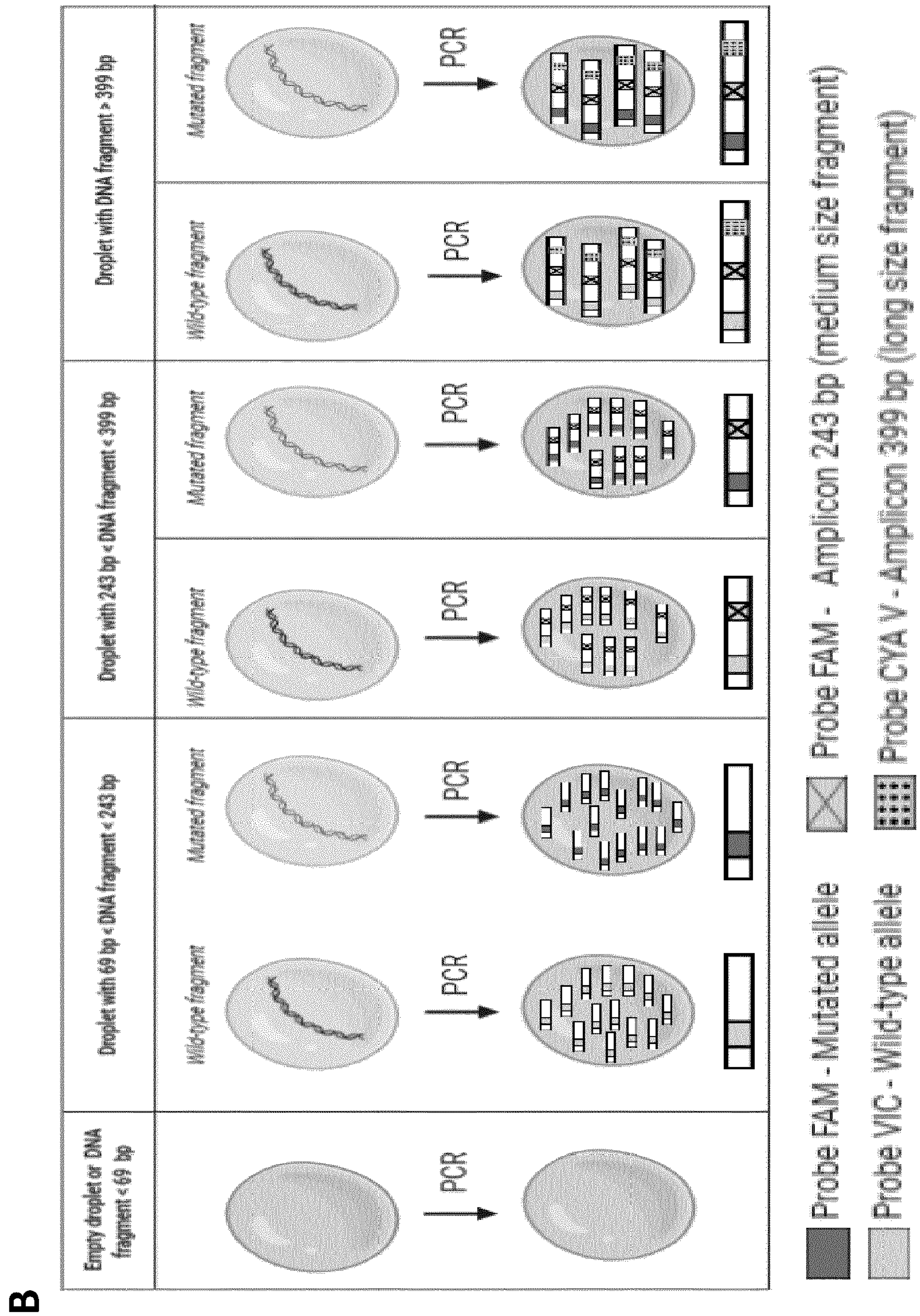


**Figure 1 (cont.)**

C

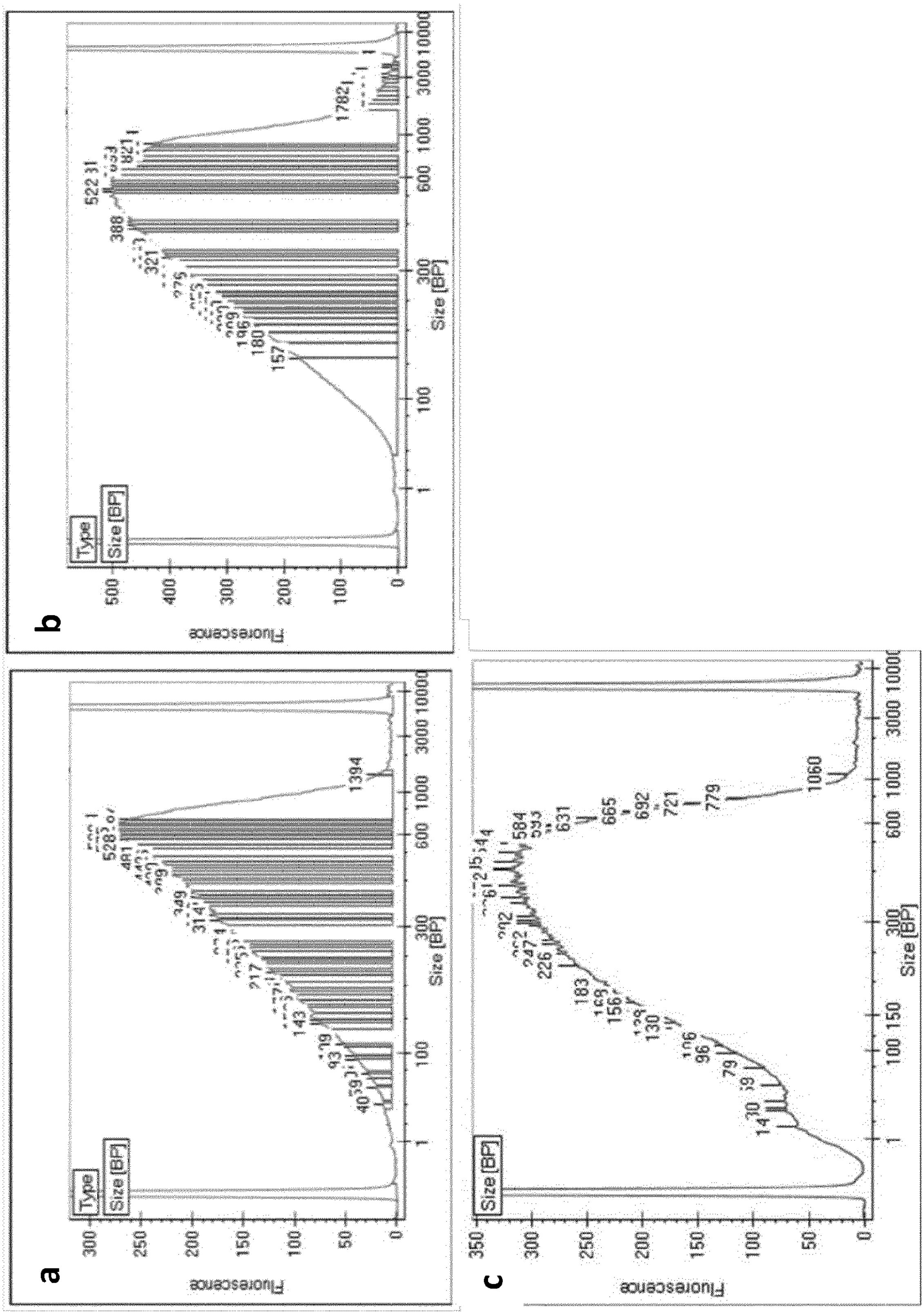


Figure 1 (cont.)

A

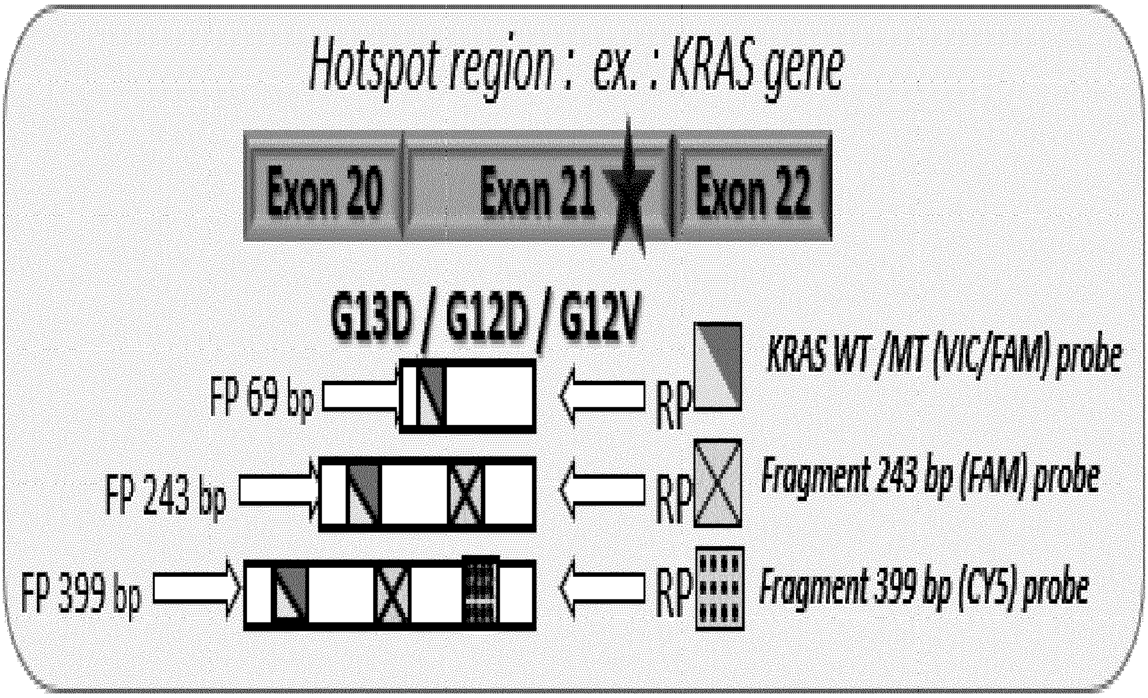
Hotspot region : ex. : KRAS gene
Exon 20
Exon 21
Exon 22
G13D / G12D / G12V
FP 69 bp
FP 243 bp
FP 399 bp
RP
RP
RP
KRAS WT /MT (VIC/FAM) probe
Fragment 243 bp (FAM) probe
Fragment 399 bp (CY5) probe

Figure 2

B

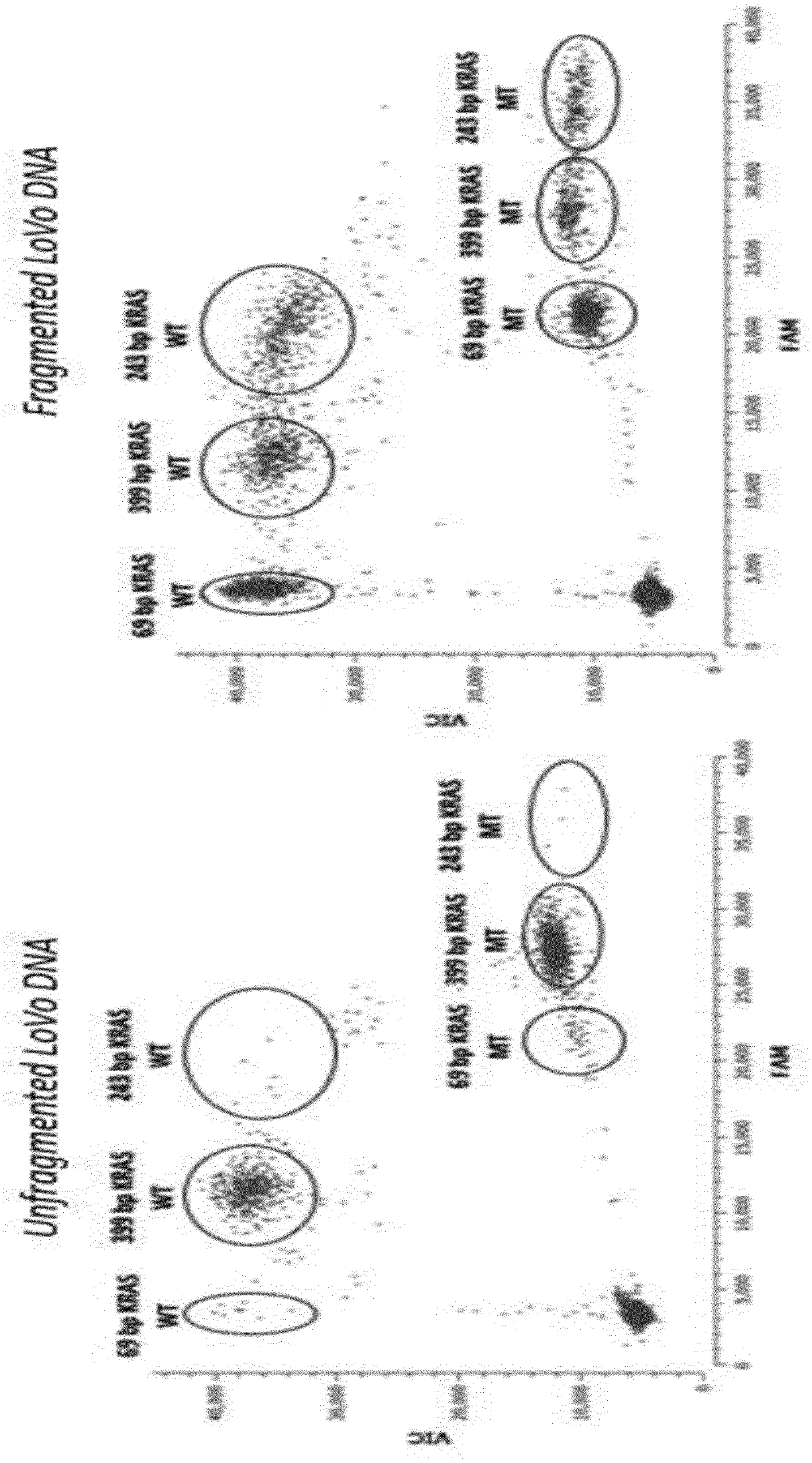


Figure 2 (cont.)

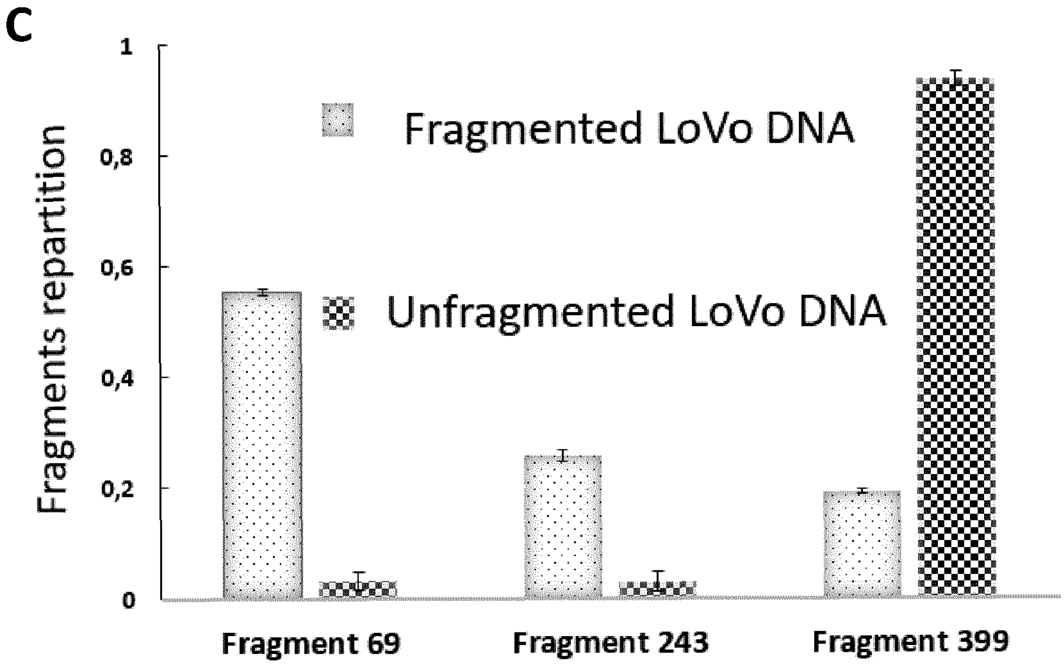


**Figure 2 (cont.)**

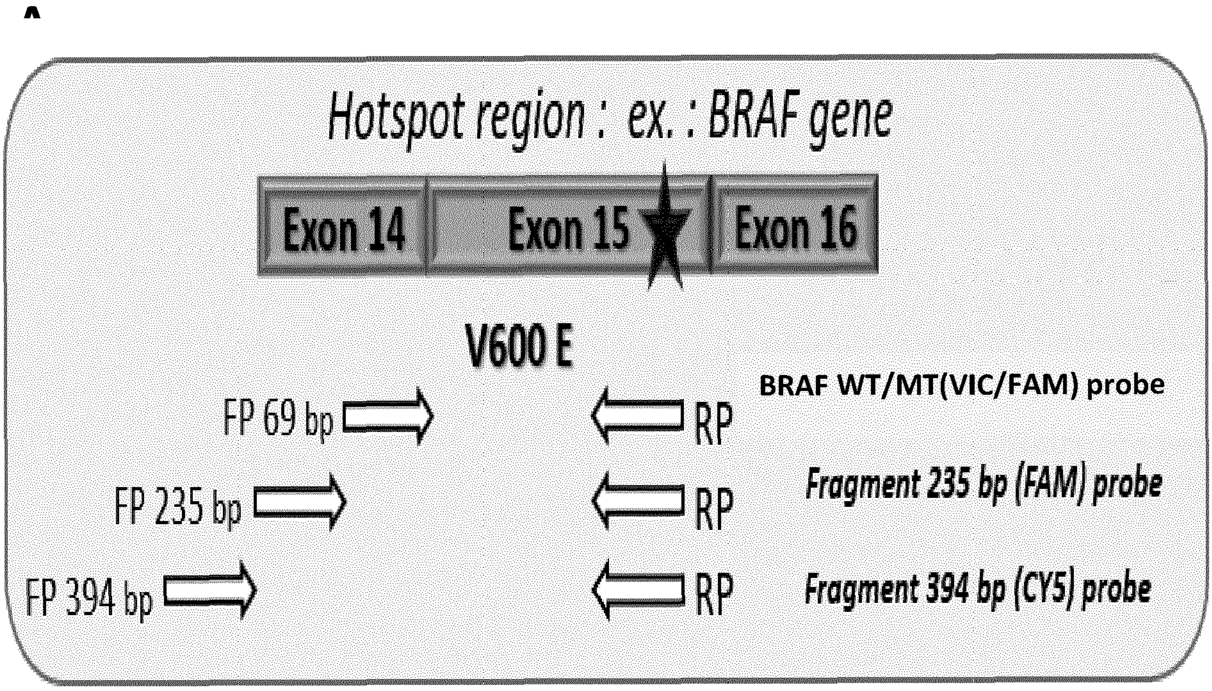


**Figure 3**

B

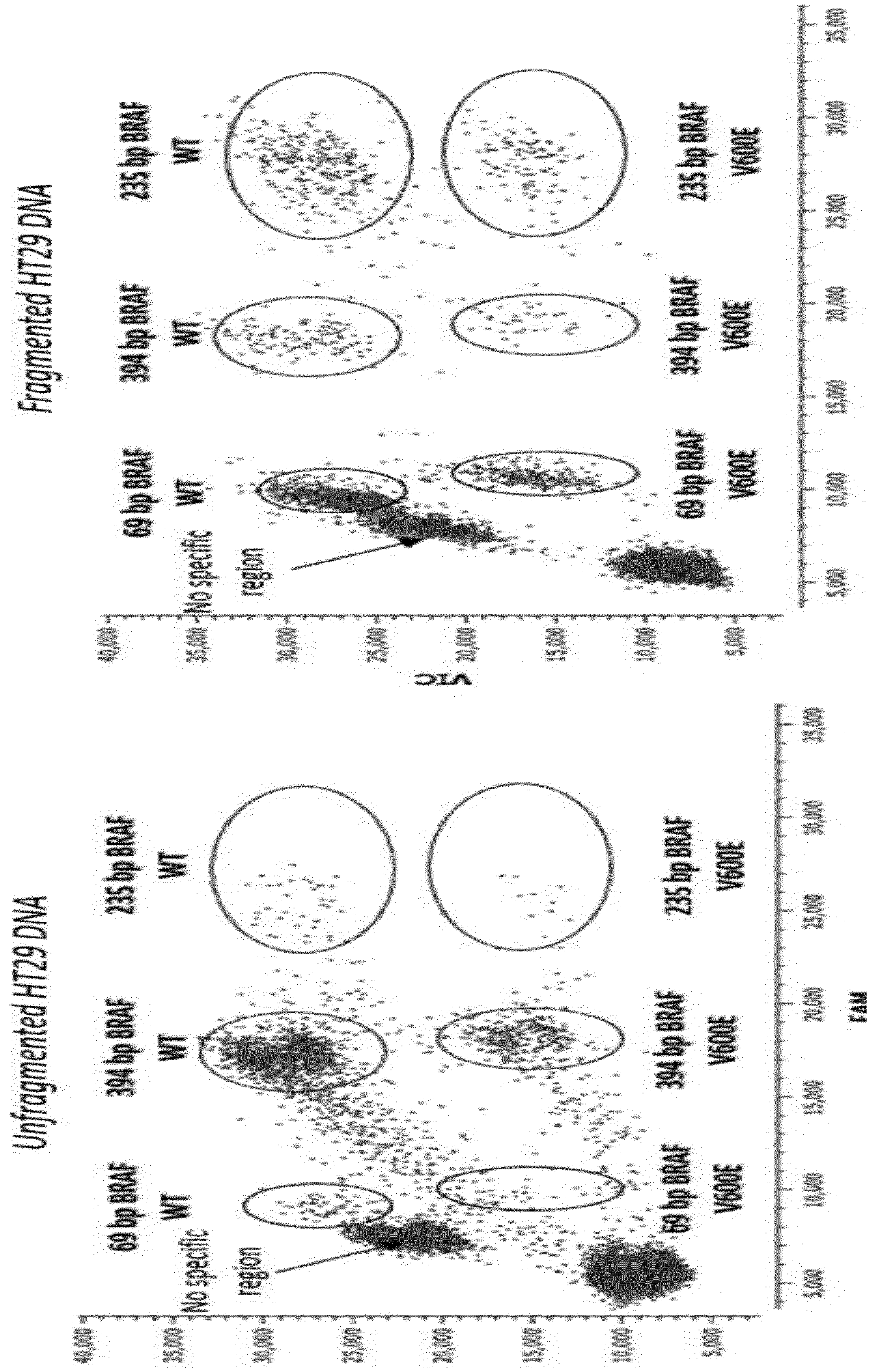


Figure 3 (cont.)

C

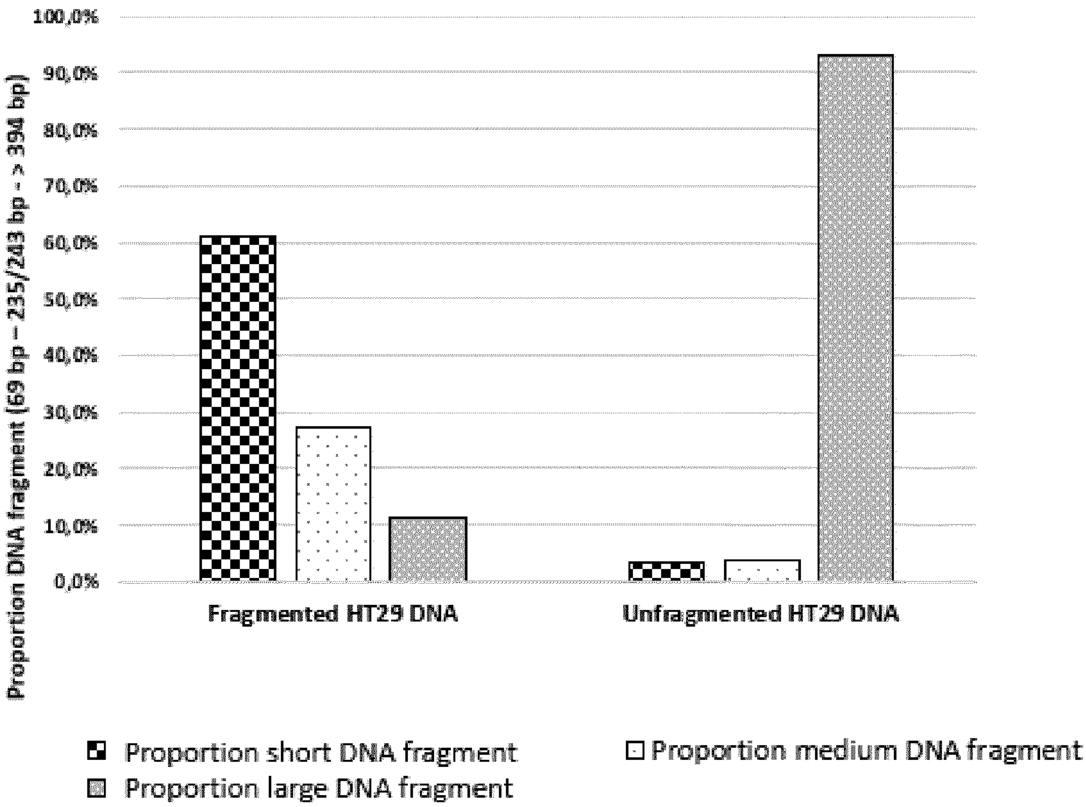


**Figure 3 (cont.)**

A

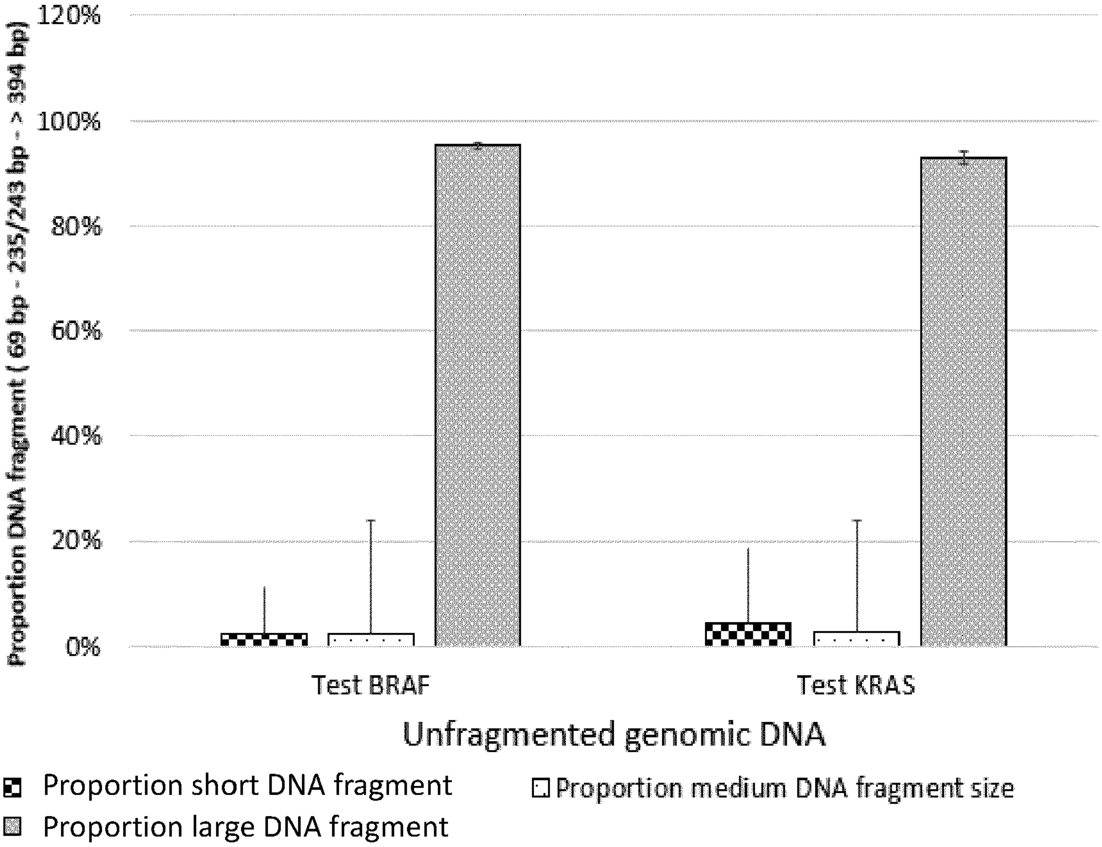
Proportion DNA fragment ( 69 bp - 235/243 bp - > 394 bp)
120%
100%
80%
60%
40%
20%
0%
Test BRAF
Test KRAS
Unfragmented genomic DNA
Proportion short DNA fragment
Proportion medium DNA fragment size
Proportion large DNA fragment

**Figure 4**

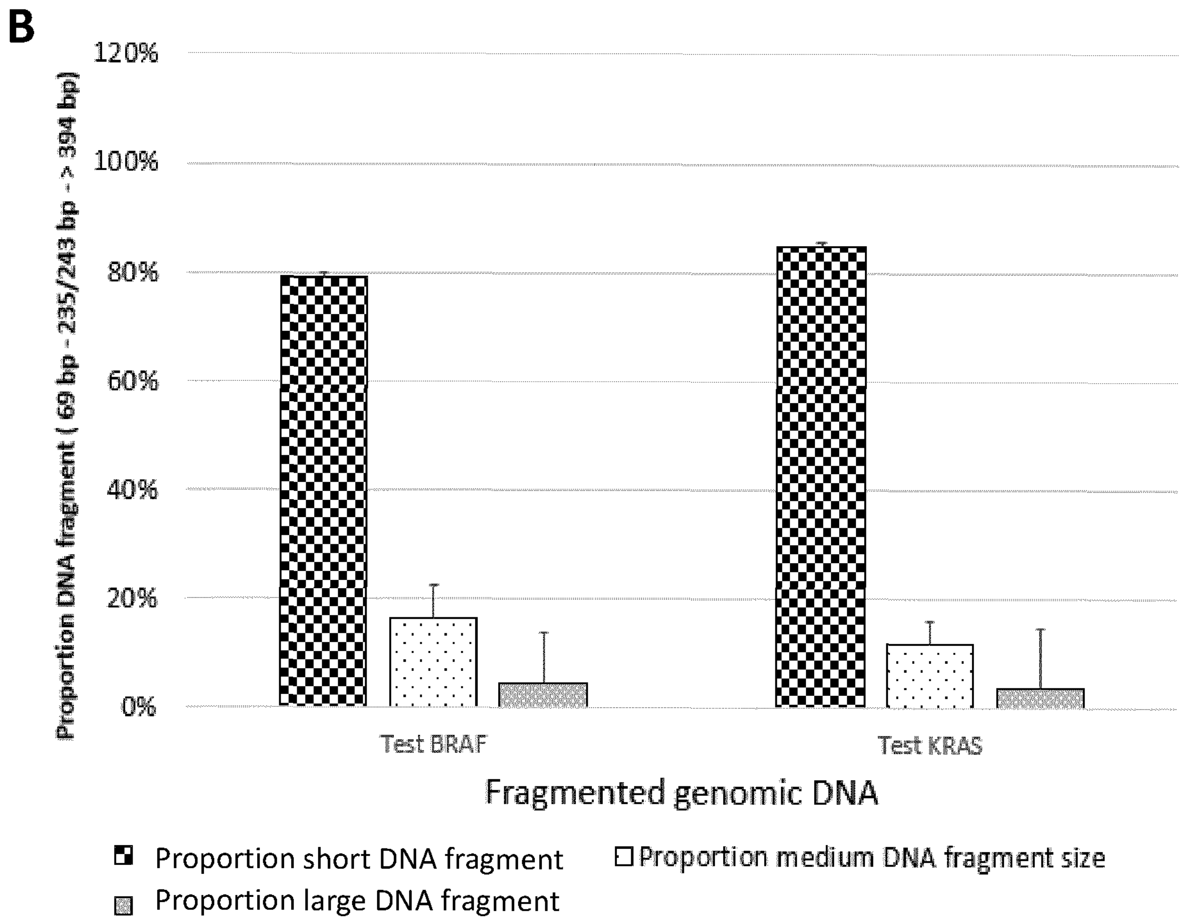


**Figure 4 (cont.)**

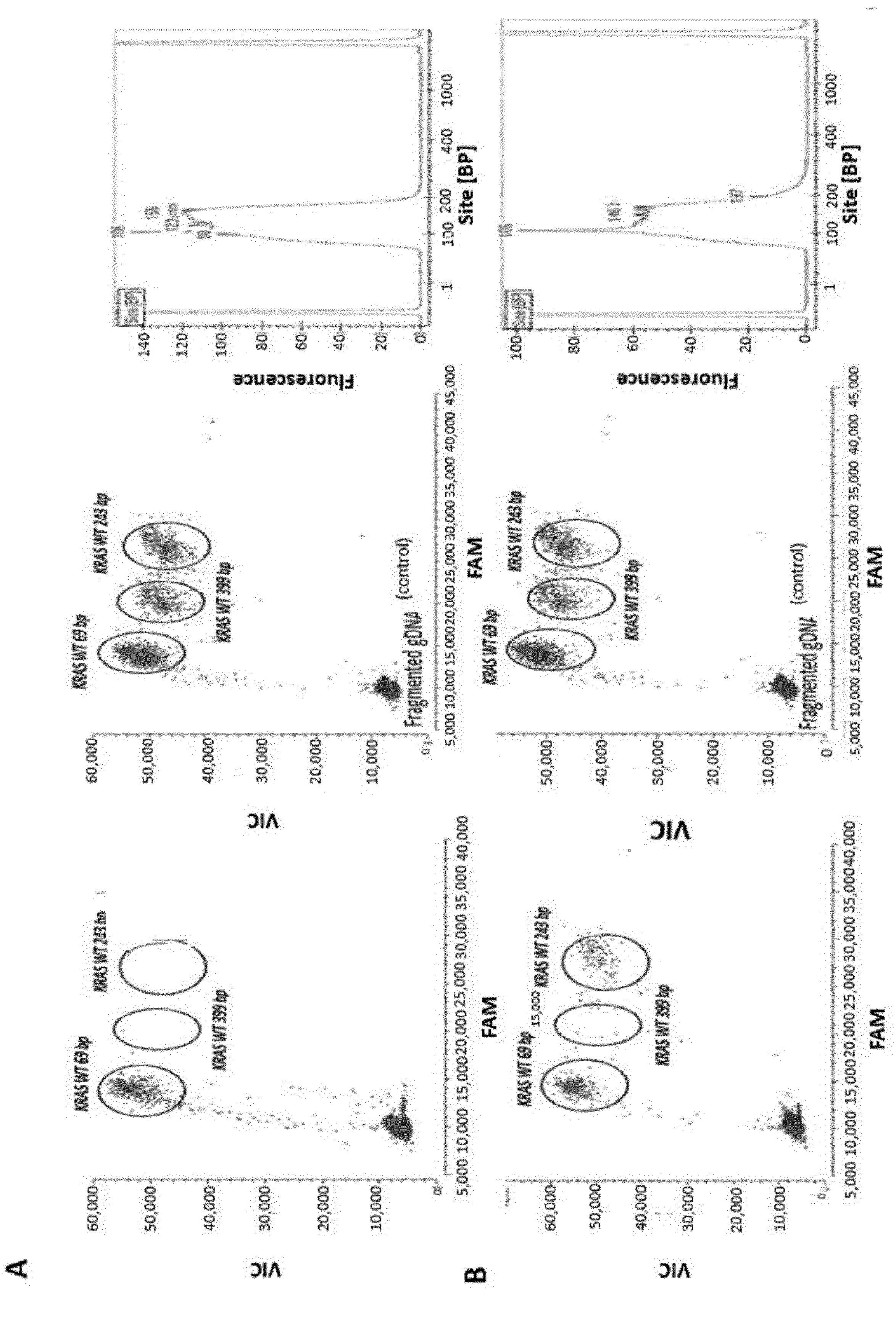
A
B
VIC
FAM
KRAS WT 69 bp
KRAS WT 399 bp
KRAS WT 243 bp
Fragmented gDNA
(control)
Fluorescence
Site [BP]

Figure 5

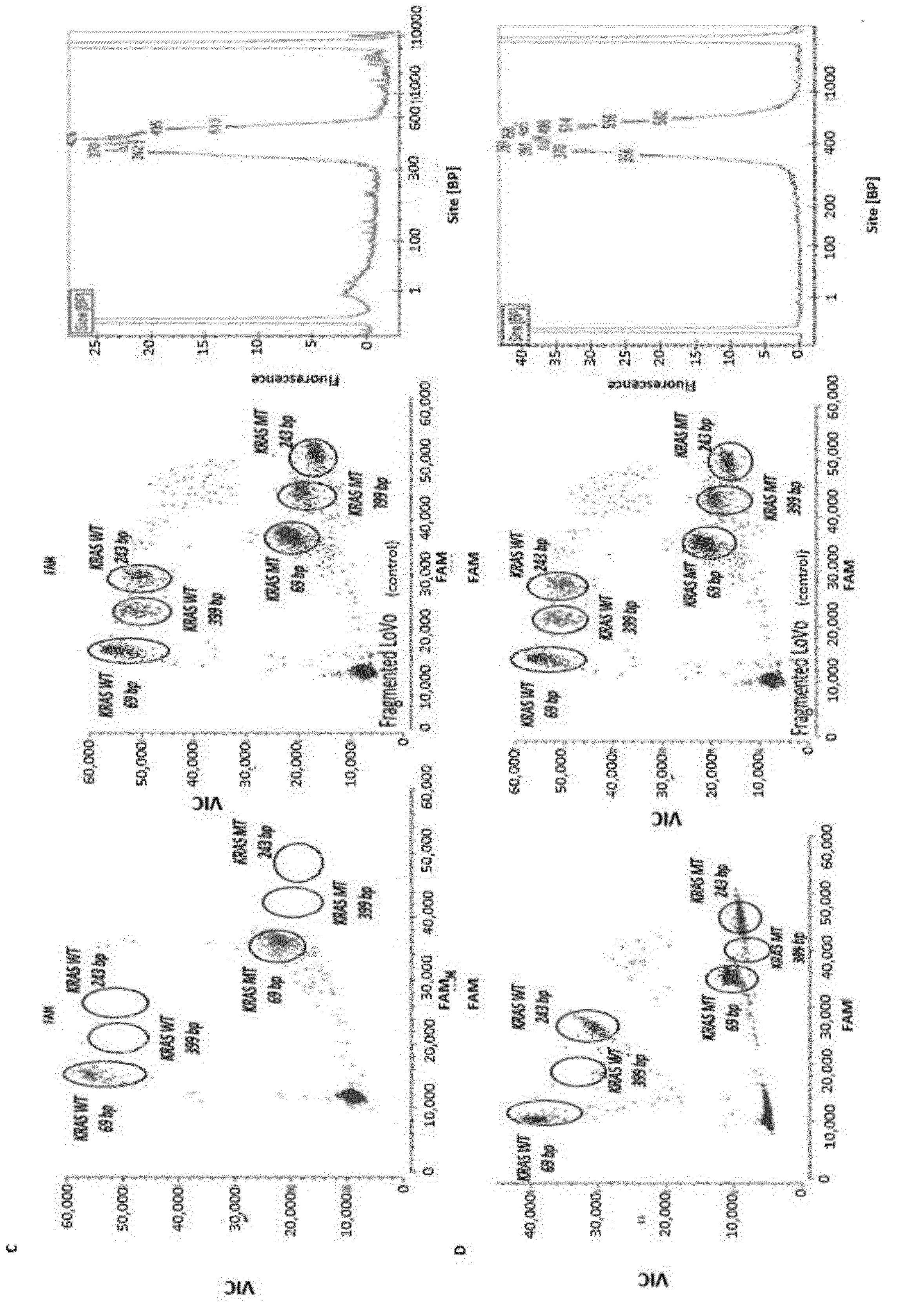


Figure 5 (cont.)

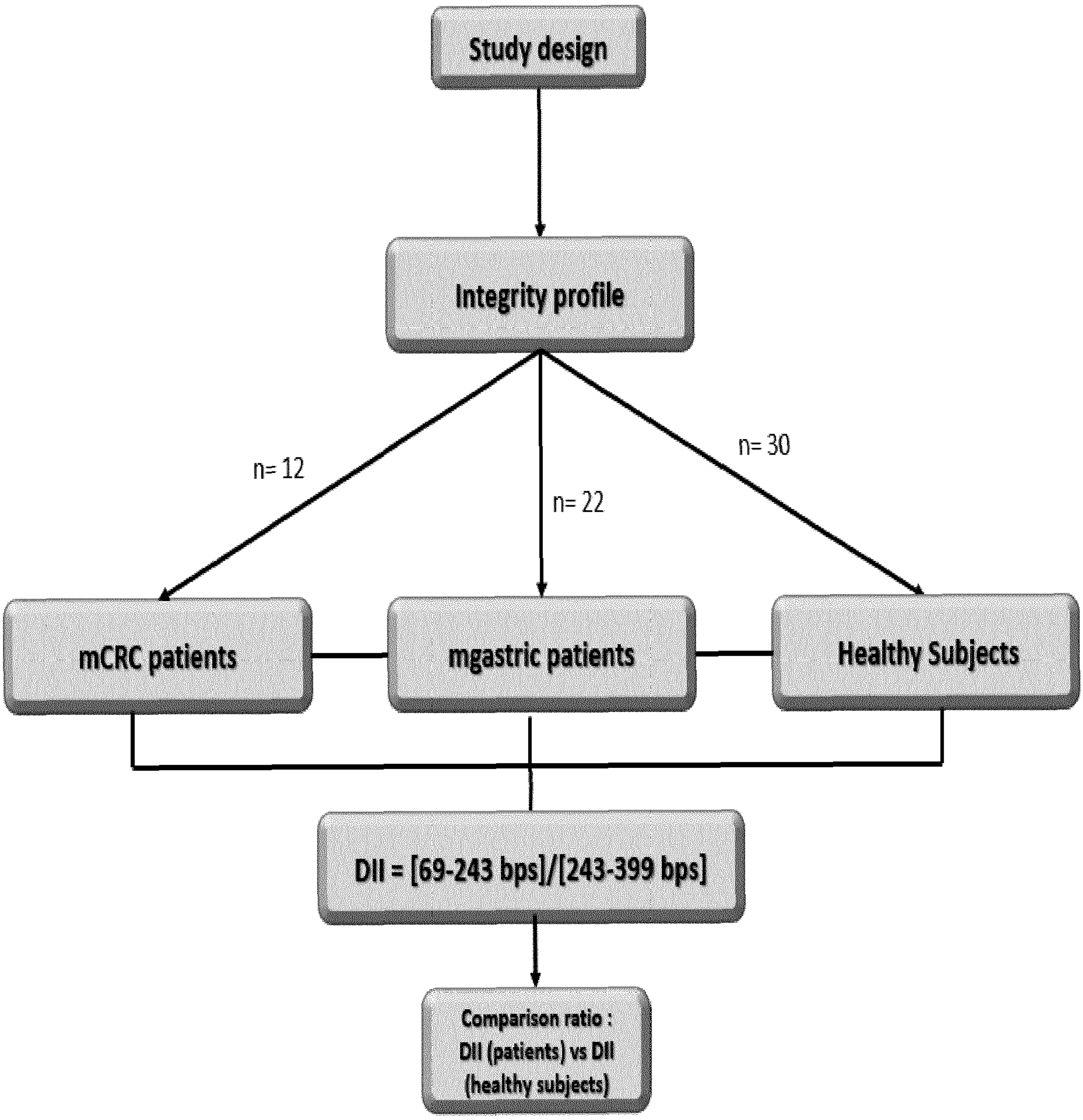
Study design
Integrity profile
n= 12
n= 22
n= 30
mCRC patients
mgastric patients
Healthy Subjects
DII = [69-243 bps]/[243-399 bps]
Comparison ratio :
DII (patients) vs DII
(healthy subjects)

Figure 6

A

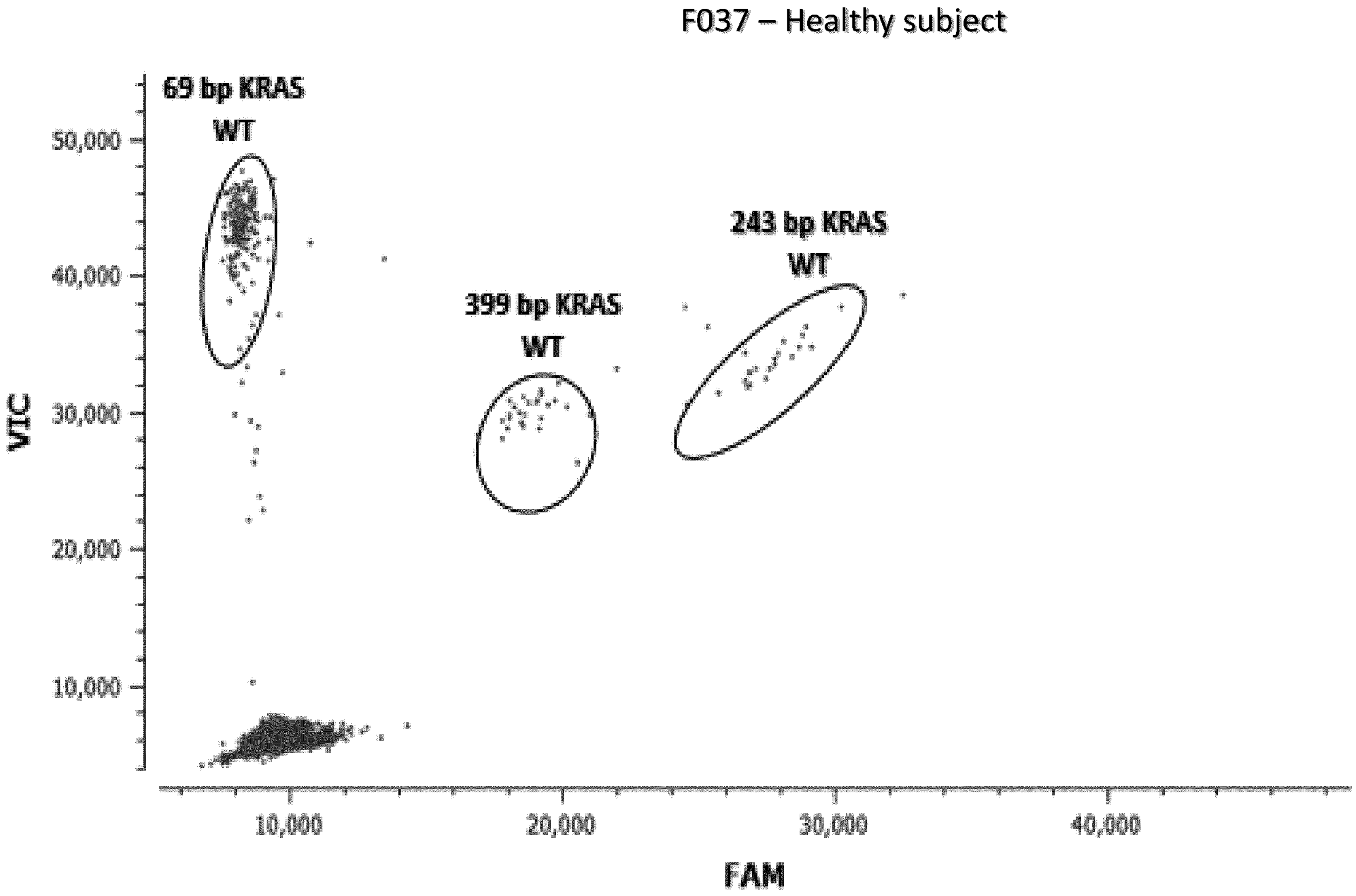
F037 – Healthy subject
69 bp KRAS
WT
399 bp KRAS
WT
243 bp KRAS
WT
VIC
50,000
40,000
30,000
20,000
10,000
10,000
20,000
30,000
40,000
FAM

Figure 7

B

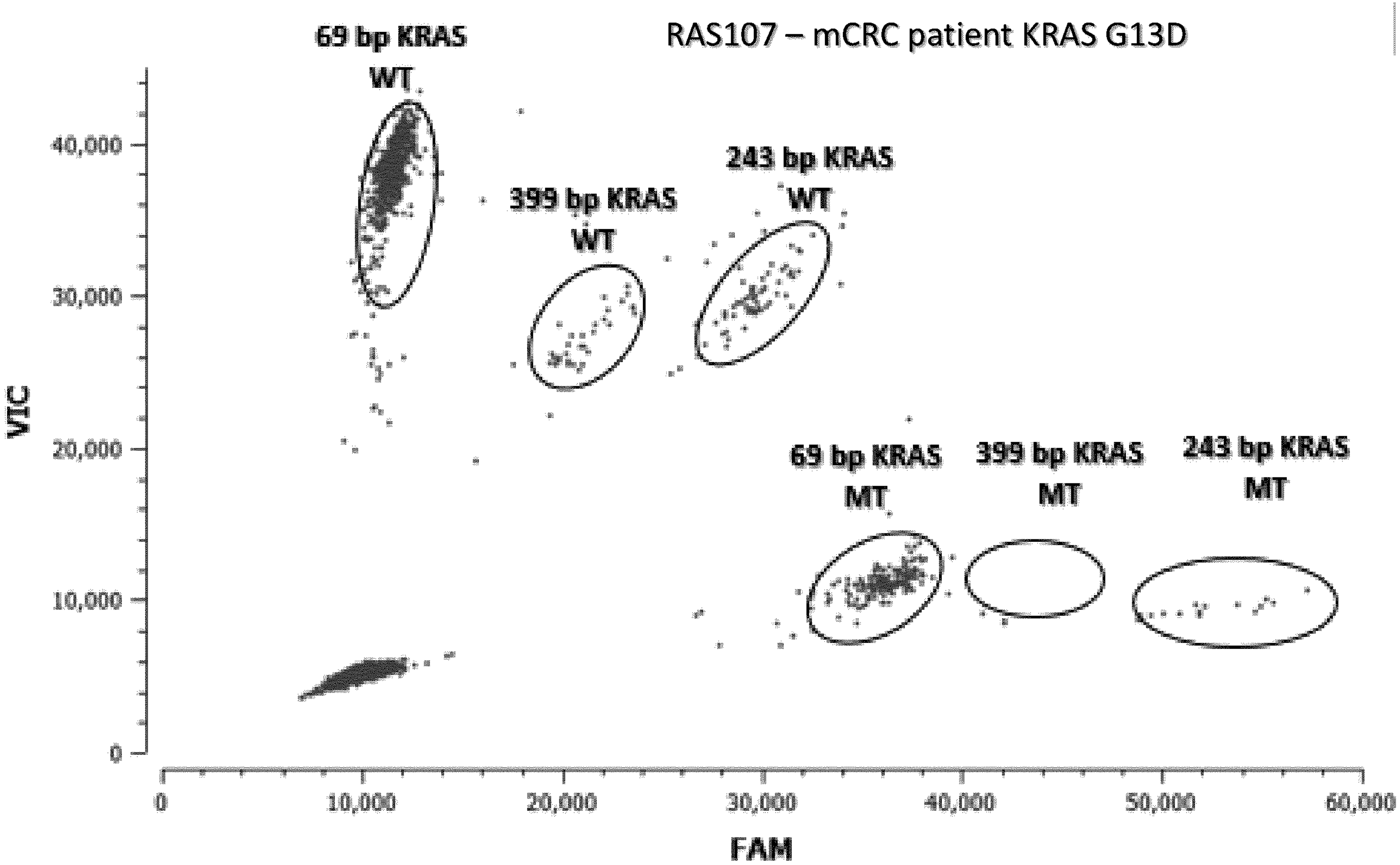


Figure 7 (cont.)

C

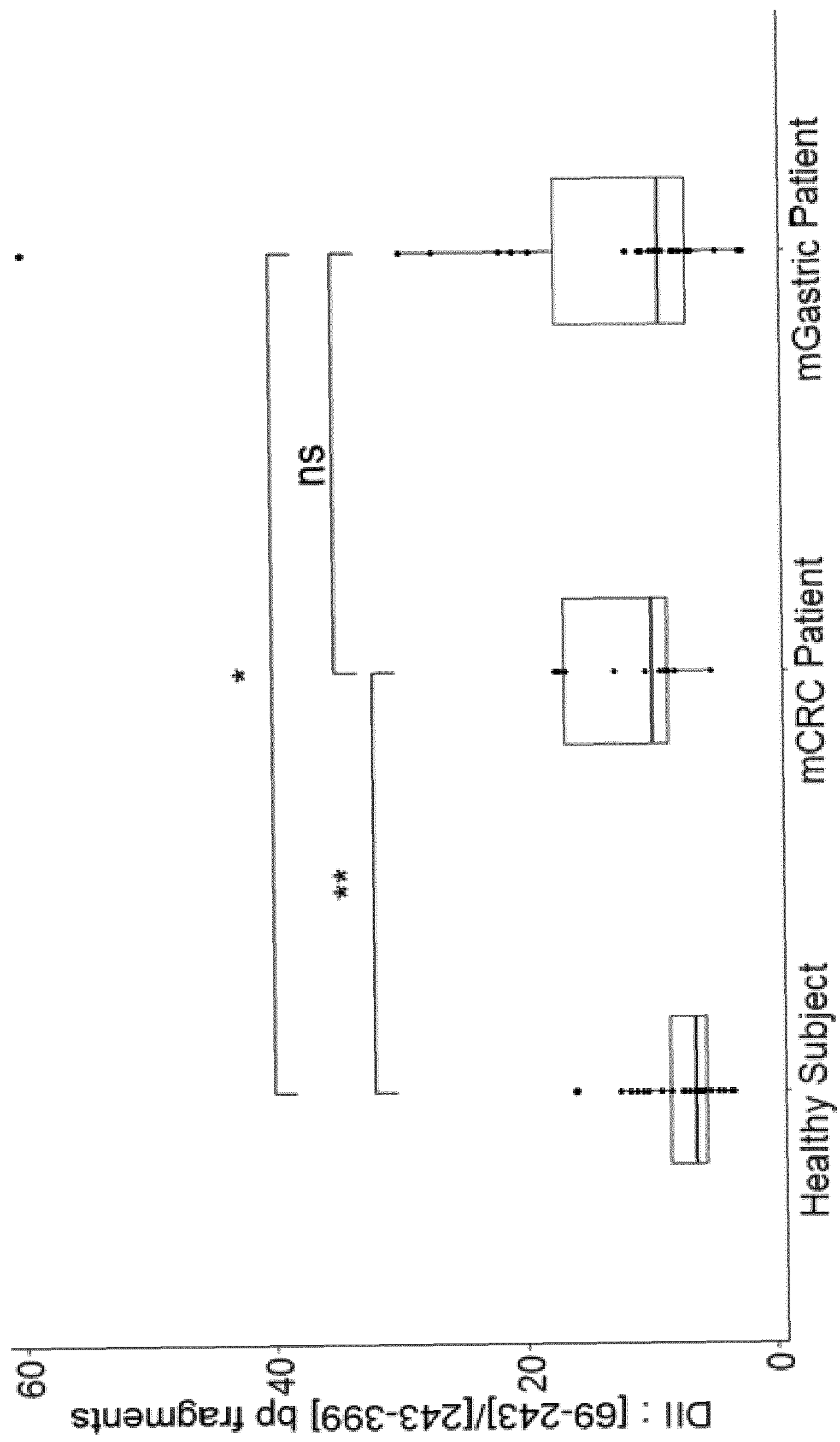


**Figure 7 (cont.)**

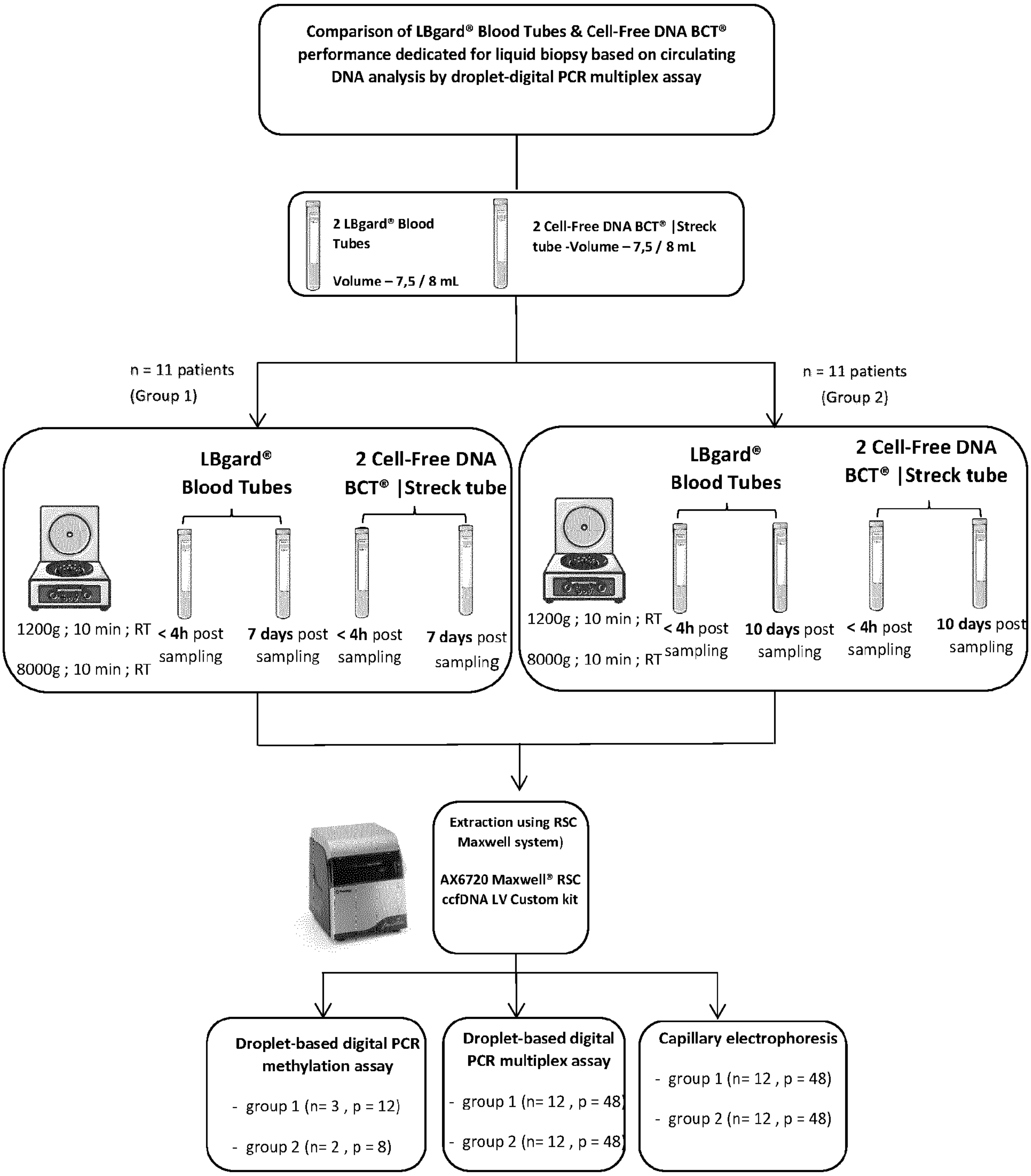
Comparison of LBgard® Blood Tubes & Cell-Free DNA BCT® performance dedicated for liquid biopsy based on circulating DNA analysis by droplet-digital PCR multiplex assay
2 LBgard® Blood Tubes
Volume – 7,5 / 8 mL
2 Cell-Free DNA BCT® |Streck tube -Volume – 7,5 / 8 mL
n = 11 patients (Group 1)
n = 11 patients (Group 2)
LBgard® Blood Tubes
2 Cell-Free DNA BCT® |Streck tube
1200g ; 10 min ; RT
8000g ; 10 min ; RT
< 4h post sampling
7 days post sampling
< 4h post sampling
7 days post sampling
LBgard® Blood Tubes
2 Cell-Free DNA BCT® |Streck tube
1200g ; 10 min ; RT
8000g ; 10 min ; RT
< 4h post sampling
10 days post sampling
< 4h post sampling
10 days post sampling
Extraction using RSC Maxwell system)
AX6720 Maxwell® RSC ccfDNA LV Custom kit
Droplet-based digital PCR methylation assay
- group 1 (n= 3 , p = 12)
- group 2 (n= 2 , p = 8)
Droplet-based digital PCR multiplex assay
- group 1 (n= 12 , p = 48)
- group 2 (n= 12 , p = 48)
Capillary electrophoresis
- group 1 (n= 12 , p = 48)
- group 2 (n= 12 , p = 48)

# Figure 8

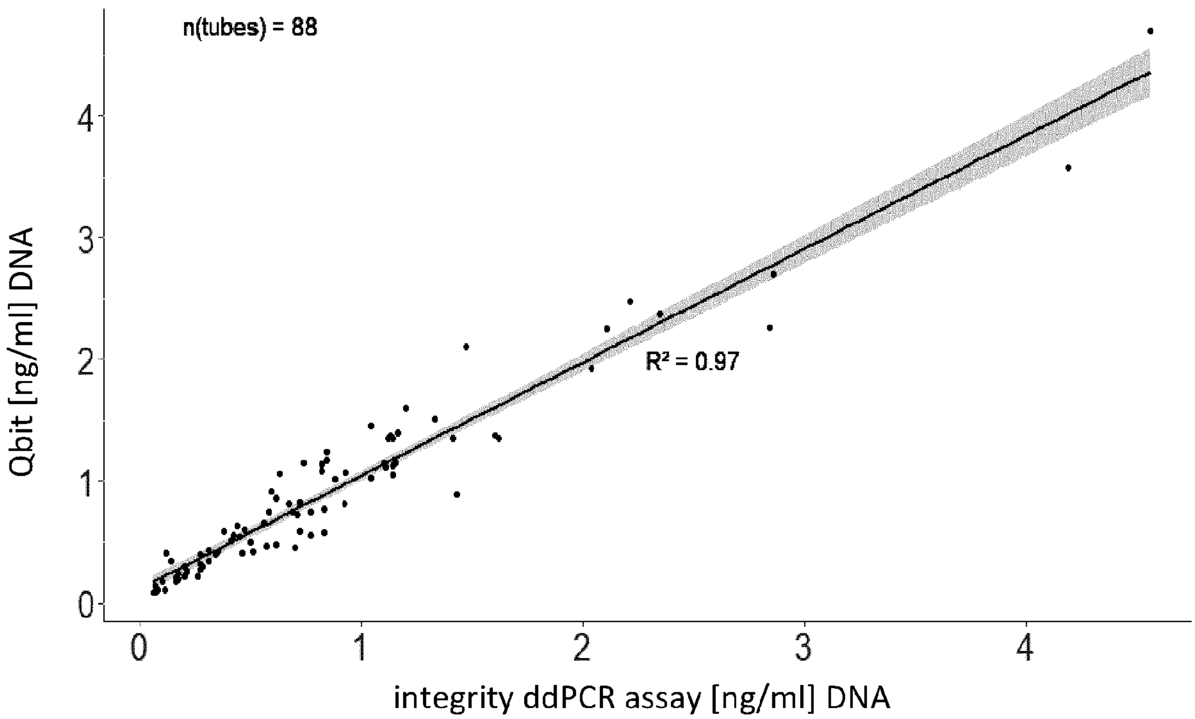


**Figure 9**

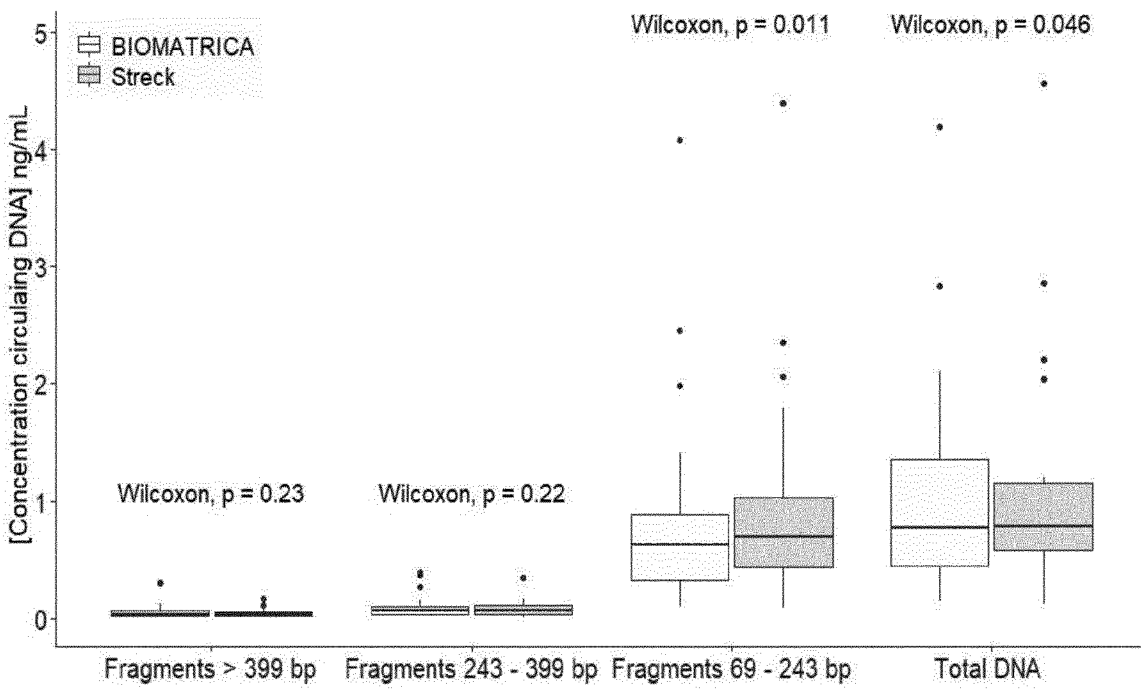


**Figure 10**

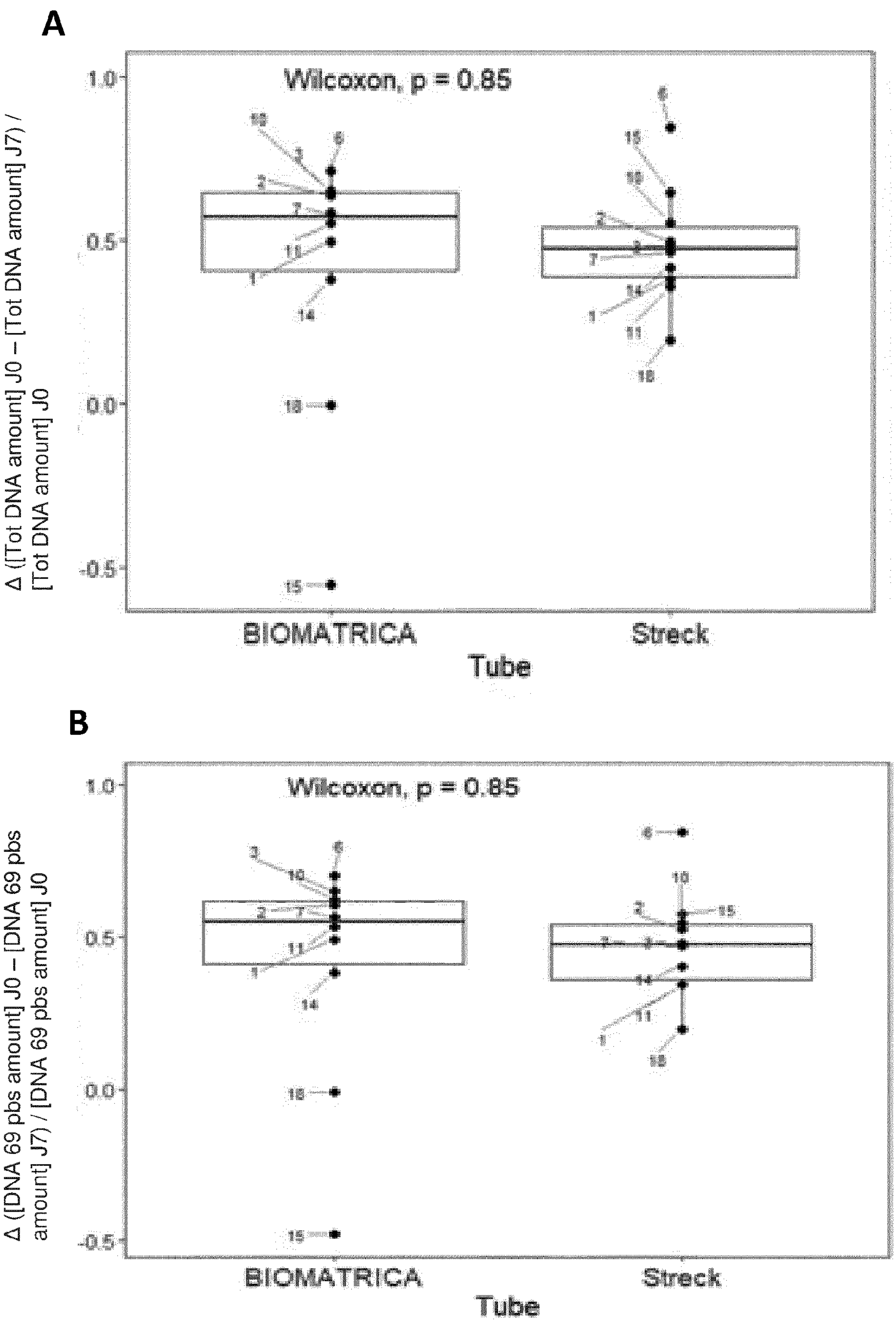

A
Wilcoxon, p = 0.85
Δ ([Tot DNA amount] J0 – [Tot DNA amount] J7) / [Tot DNA amount] J0
1.0
0.5
0.0
-0.5
BIOMATRICA
Streck
Tube
B
Wilcoxon, p = 0.85
Δ ([DNA 69 pbs amount] J0 – [DNA 69 pbs amount] J7) / [DNA 69 pbs amount] J0
1.0
0.5
0.0
-0.5
BIOMATRICA
Streck
Tube


**Figure 11**

C

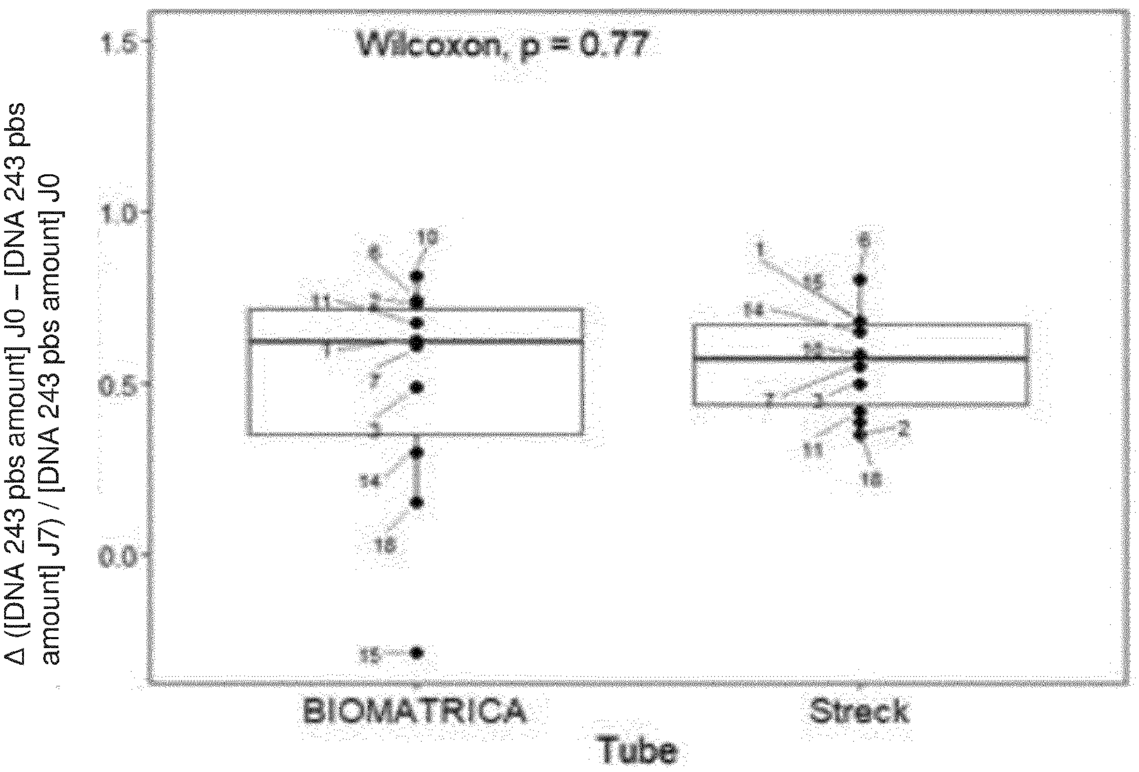


D

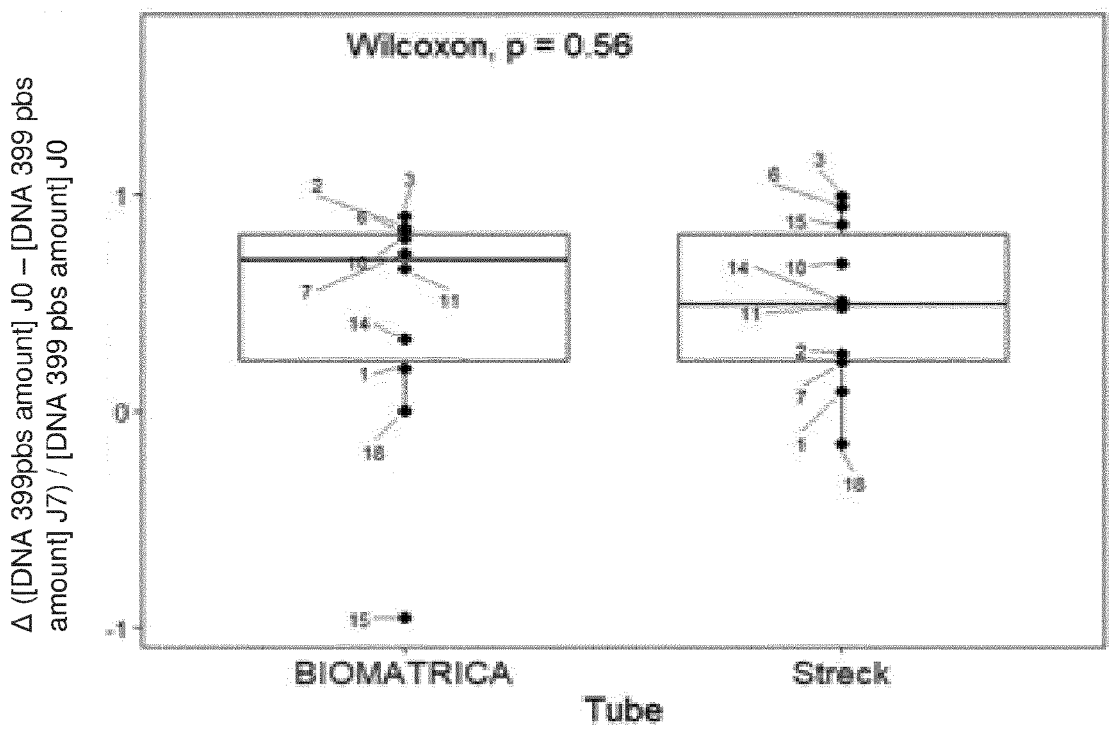


**Figure 11 (cont.)**

A

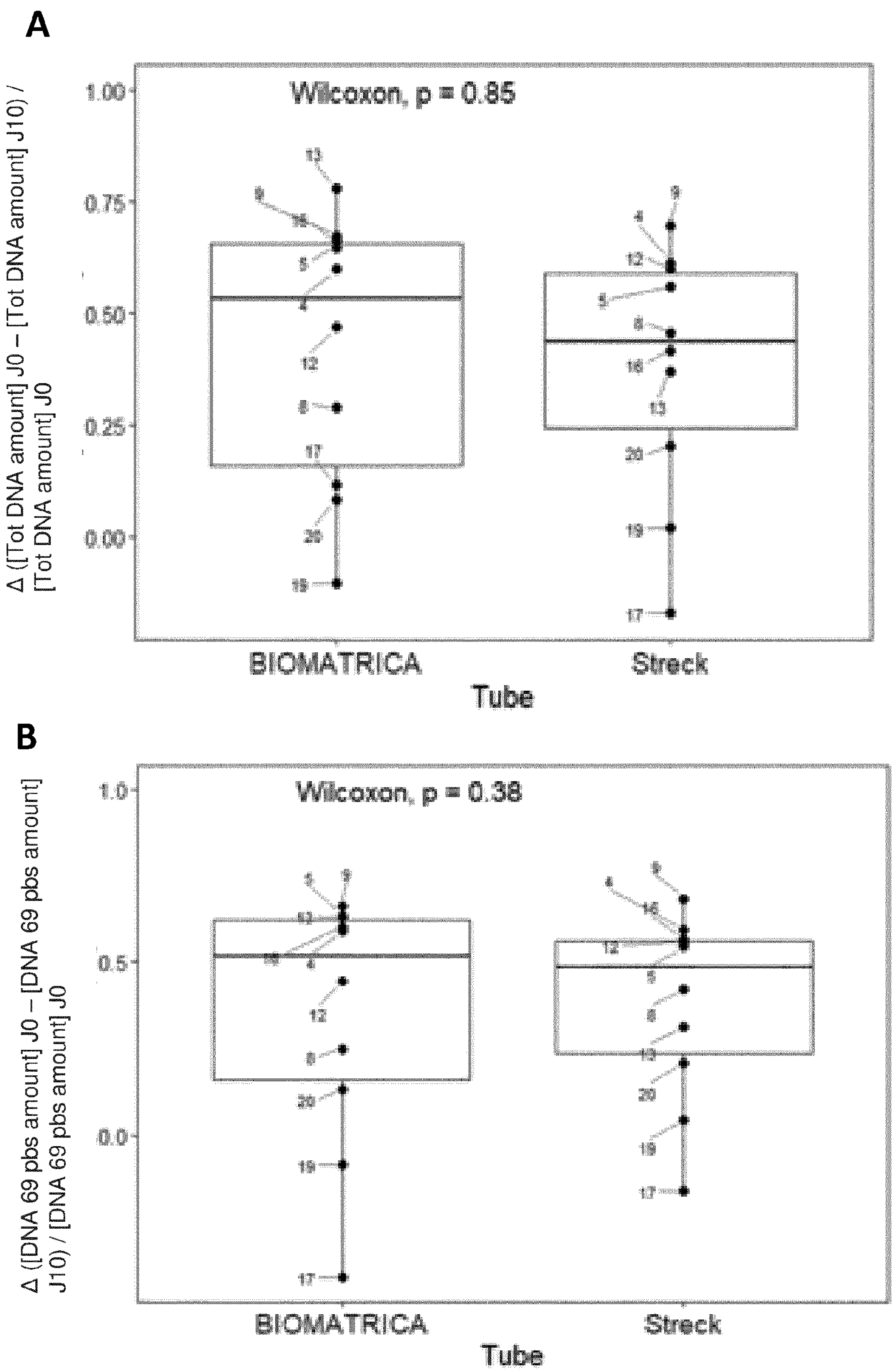
A
Wilcoxon, p = 0.85
1.00
0.75
0.50
0.25
0.00
Δ ([Tot DNA amount] J0 – [Tot DNA amount] J10) / [Tot DNA amount] J0
BIOMATRICA
Streck
Tube
B
Wilcoxon, p = 0.38
1.0
0.5
0.0
Δ ([DNA 69 pbs amount] J0 – [DNA 69 pbs amount] J10) / [DNA 69 pbs amount] J0
BIOMATRICA
Streck
Tube

Figure 12

C

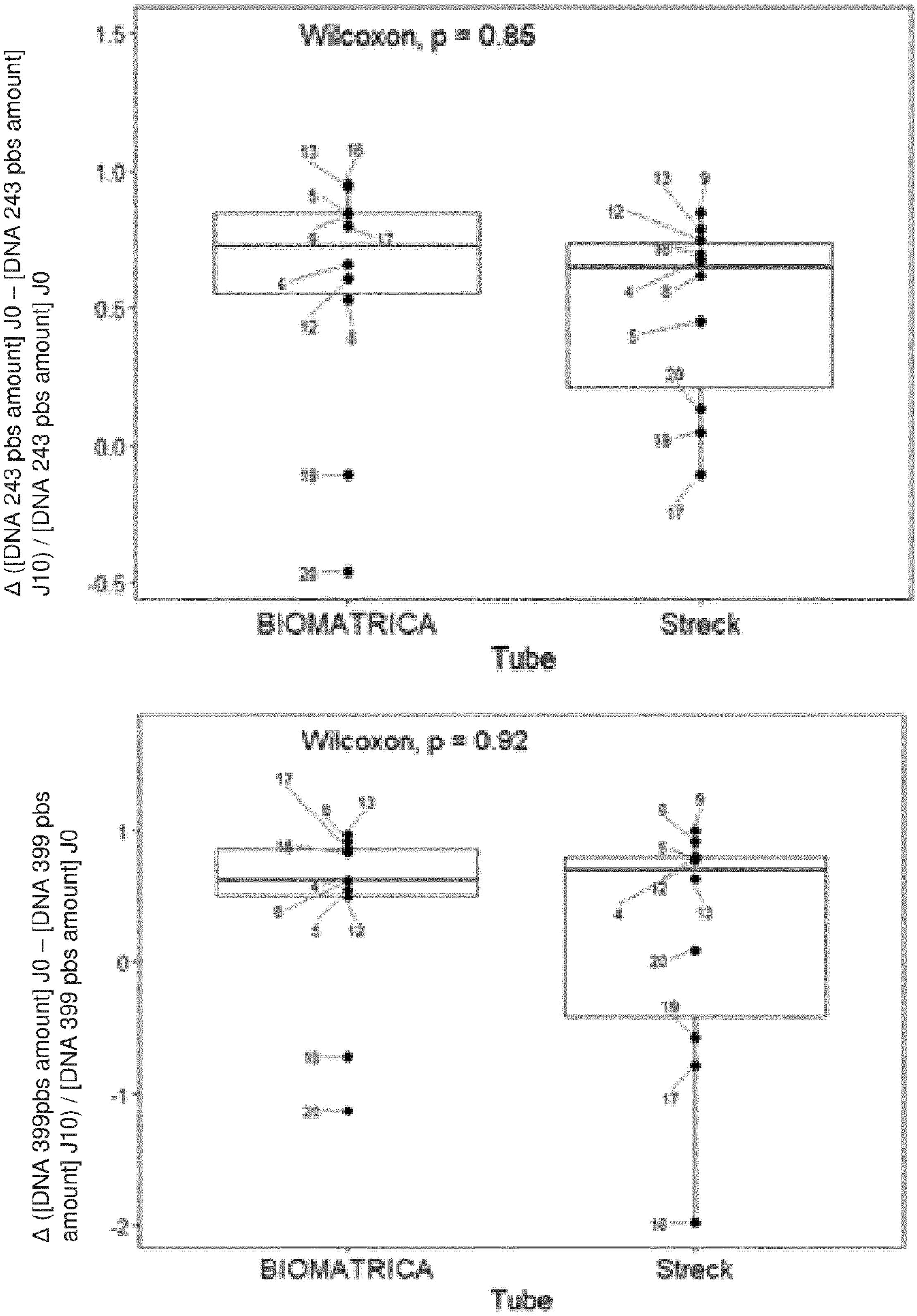


**Figure 12 (cont.)**

Europäisches Patentamt
European Patent Office
Office européen des brevets

# EUROPEAN SEARCH REPORT

Application Number
EP 20 30 6413

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 2009/019455 A2 (UNIV HONG KONG CHINESE [CN]; LO YUK MING DENNIS [CN] ET AL.) 12 February 2009 (2009-02-12)<br>* the whole document *<br>* para. 11, 13, 25, 29, 33, 35, 37-38, 41, 46, 87, Fig. 1-2 *<br>----- | 1-3,5,6, 8-15<br><br>4,7 | INV.<br>C12Q1/686<br>C12Q1/6886 |
| X<br><br>Y | EP 2 986 762 A2 (RAINDANCE TECHNOLOGIES INC [US]) 24 February 2016 (2016-02-24)<br>* the whole document *<br>* para. 1, 7-9, 25, 29, 194, 195, 214.216, 219, 221-223, 227-228 *<br>----- | 1-3,5,6, 8-15<br>4,7 | |
| X<br><br>Y | WO 2016/168844 A1 (THE TRANSLATIONAL GENOMICS RES INST [US]) 20 October 2016 (2016-10-20)<br>* the whole document *<br>* para. 5, 13, 24, 26, 41, 51, 74, Fig. 1-3, claims *<br>----- | 1-3,5,6, 8-15<br><br>4,7 | |
| X<br><br>Y | US 2005/042639 A1 (KNAPP MICHAEL R [US] ET AL) 24 February 2005 (2005-02-24)<br>* the whole document *<br>* para. 53, 55-56, 235, 239, 241, Fig. 15 *<br>----- | 1-3,5,6, 8-12,14<br>4,7,13, 15 | TECHNICAL FIELDS SEARCHED (IPC)<br>C12Q |
| Y | CN 107 254 541 B (GUANGZHOU MEDEL GENE MEDICAL TECH CO LTD) 8 May 2020 (2020-05-08)<br>* the whole document *<br>* para. 26, 78, claims *<br>----- | 1-15 | |
| Y | US 2003/017482 A1 (GODFREY TONY E [US] ET AL) 23 January 2003 (2003-01-23)<br>* the whole document *<br>* para. 57-61, claim 30 *<br>----- | 1-15 | |

The present search report has been drawn up for all claims

1

| Place of search | Date of completion of the search | Examiner |
|---|---|---|
| Munich | 4 May 2021 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 30 6413

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2021

| Patent document cited in search report | Publication date | Patent family member(s) | Publication date |
|---|---|---|---|
| WO 2009019455 A2 | 12-02-2009 | AU 2008285465 A1 | 12-02-2009 |
| | | CA 2694619 A1 | 12-02-2009 |
| | | CN 101855363 A | 06-10-2010 |
| | | EP 2183387 A2 | 12-05-2010 |
| | | KR 20100063050 A | 10-06-2010 |
| | | US 2009053719 A1 | 26-02-2009 |
| | | US 2011183330 A1 | 28-07-2011 |
| | | WO 2009019455 A2 | 12-02-2009 |
| EP 2986762 A2 | 24-02-2016 | EP 2986762 A2 | 24-02-2016 |
| | | WO 2014172288 A2 | 23-10-2014 |
| WO 2016168844 A1 | 20-10-2016 | EP 3283657 A1 | 21-02-2018 |
| | | US 2018105864 A1 | 19-04-2018 |
| | | US 2020283827 A1 | 10-09-2020 |
| | | WO 2016168844 A1 | 20-10-2016 |
| US 2005042639 A1 | 24-02-2005 | AU 2005245448 A1 | 01-12-2005 |
| | | CA 2566698 A1 | 01-12-2005 |
| | | EP 1755785 A1 | 28-02-2007 |
| | | JP 4740237 B2 | 03-08-2011 |
| | | JP 2008502325 A | 31-01-2008 |
| | | US 2005042639 A1 | 24-02-2005 |
| | | US 2008085521 A1 | 10-04-2008 |
| | | WO 2005113148 A1 | 01-12-2005 |
| CN 107254541 B | 08-05-2020 | NONE | |
| US 2003017482 A1 | 23-01-2003 | CA 2439402 A1 | 12-09-2002 |
| | | CN 1500151 A | 26-05-2004 |
| | | EP 1373569 A1 | 02-01-2004 |
| | | JP 4808365 B2 | 02-11-2011 |
| | | JP 2004528029 A | 16-09-2004 |
| | | JP 2008271980 A | 13-11-2008 |
| | | JP 2010162042 A | 29-07-2010 |
| | | US 2003017482 A1 | 23-01-2003 |
| | | US 2006205006 A1 | 14-09-2006 |
| | | WO 02070751 A1 | 12-09-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MANIATIS et al.** Molecular Cloning: A Laboratory Manual. Cold Spring Harbor, 1982, 280-281
- **HINDSON et al.** *Analytical Chemistry,* 2011, vol. 83, 8604-8610
- **PEKIN et al.** *Lab on a Chip,* 2011, vol. 11, 2156-66
- **PINHEIRO et al.** *Anal Chem,* 2012, vol. 84, 1003-1011

SEQUENCE LISTING

<110> Université de Paris et al

<120> A method for determining the level of DNA integrity

<130> PR2532

<160> 16

<170> PatentIn version 3.5

<210> 1
<211> 17
<212> DNA
<213> Artificial Sequence

<220>
<223> Probe sequence for KRAS exon 2, codon 13 (WT sequence)

<400> 1
aggtttttta taattta 17

<210> 2
<211> 14
<212> DNA
<213> Artificial Sequence

<220>
<223> Probe sequence for KRAS exon 2 codon 13 (mutated sequence)

<400> 2
cggcgttagg ttgc 14

<210> 3
<211> 18
<212> DNA
<213> Artificial Sequence

<220>
<223> Probe sequence for KRAS, exon 2, Fragment 243 bp

<400> 3
aaggtactgg tggagtat 18

<210> 4
<211> 16
<212> DNA
<213> Artificial Sequence

<220>
<223> Probe sequence for KRAS, exon 2, Fragment 399bp

<400> 4
acgtctgcag tcaact 16

<210> 5
<211> 28
<212> DNA

<213> Artificial Sequence

<220>
<223> Forward primer sequence for KRAS, exon 2, fragment 69 bp

<400> 5
gaaaatgact gaatataaac ttgtggta 28

<210> 6
<211> 20
<212> DNA
<213> Artificial Sequence

<220>
<223> Forward primer sequence for KRAS, exon 2, Fragment 243 bp

<400> 6
gcagtcaact ggaattttca 20

<210> 7
<211> 16
<212> DNA
<213> Artificial Sequence

<220>
<223> Forward primer sequence for KRAS, exon 2, Fragment 399bp

<400> 7
tgagagcctt tagccg 16

<210> 8
<211> 18
<212> DNA
<213> Artificial Sequence

<220>
<223> Reverse primer sequence for KRAS exon 2

<400> 8
tgtatcgtca aggcactc 18

<210> 9
<211> 20
<212> DNA
<213> Artificial Sequence

<220>
<223> Probe sequence for BRAF exon 15, codon 600 (WT sequence)

<400> 9
ggtctagcta cagagaaatc 20

<210> 10
<211> 19
<212> DNA
<213> Artificial Sequence

<220>

<223>  Probe sequence for BRAF exon 15, codon 600  (mutated sequence)

<400>  10
ggtctagcta cagtgaaat    19

<210>  11
<211>  17
<212>  DNA
<213>  Artificial Sequence

<220>
<223>  Probe sequence for BRAF, exon 15, Fragment 235 bp

<400>  11
tgcttgctct gatagga    17

<210>  12
<211>  18
<212>  DNA
<213>  Artificial Sequence

<220>
<223>  Probe sequence for BRAF , exon 15, Fragment 394 bp

<400>  12
ttaggaaagc atctcacc    18

<210>  13
<211>  25
<212>  DNA
<213>  Artificial Sequence

<220>
<223>  Forward primer sequence for BRAF, exon 15, fragment 69 bp

<400>  13
gacctcacag taaaaatagg tgatt    25

<210>  14
<211>  22
<212>  DNA
<213>  Artificial Sequence

<220>
<223>  Forward primer sequence for BRAF, exon 15, Fragment 235 bp

<400>  14
gactctaaga ggaaagatga ag    22

<210>  15
<211>  16
<212>  DNA
<213>  Artificial Sequence

<220>
<223>  Forward primer sequence for BRAF , exon 15, Fragment 394 bp

<400>  15

```
tctgggccta cattgc                                                    16


<210>  16
<211>  17
<212>  DNA
<213>  Artificial Sequence

<220>
<223>  Reverse primer sequence for BRAF exon 15

<400>  16
ctgatgggac ccactcc                                                   17
```